# EUROPÄISCHE PATENTSCHRIFT

(11) **EP 1 993 515 B1**
(45) Veröffentlichungstag und Bekanntmachung des Hinweises auf die Patenterteilung: **29.07.2009**
(21) Anmeldenummer: 07710794.4
(22) Anmeldetag: 09.03.2007
(51) Int. Cl.: A61K 8/90, A01N 25/02, A01N 25/04, A01N 43/90, A61K 8/67, A61K 8/98, A61K 9/14, A61K 35/04, A61K 38/28, A61Q 5/02

(54) **VERFAHREN ZUM SOLUBILISIEREN, DISPERGIEREN UND STABILISIEREN VON STOFFEN, NACH DEM VERFAHREN HERGESTELLTE ERZEUGNISSE SOWIE DIE VERWENDUNG DERSELBEN**
METHOD FOR SOLUBILIZING, DISPERSING, AND STABILIZING MATERIALS, PRODUCTS MANUFACTURED ACCORDING TO SAID METHOD, AND USE THEREOF
PROCÉDÉ POUR SOLUBILISER, DISPERSER ET STABILISER DES MATIÈRES, PRODUITS RÉALISÉS PAR CE PROCÉDÉ ET LEUR UTILISATION

(30) Priorität: 10.03.2006 CH 377062006
(43) Veröffentlichungstag der Anmeldung: 26.11.2008
(73) Patentinhaber: Laboswiss AG, 7270 Davos-Platz (CH)
(72) Erfinder: STROBEL, Hanspeter, CH-7270 Davos-Platz (CH)
(74) Vertreter: Felber, Josef
(86) Internationale Anmeldenummer: PCT/CH2007/000131
(87) Internationale Veröffentlichungsnummer: WO 2007/104173

(56) Entgegenhaltungen:
- EP-A- 0 870 507
- WO-A-00/33802
- WO-A-00/54812
- WO-A-01/19329
- WO-A-98/02142
- DE-A- 10 343 872
- FR-A- 2 336 475
- FR-A- 2 806 909
- GB-A- 2 181 737
- US-A- 4 383 987
- US-A- 4 810 503
- US-A- 5 667 795
- US-A1- 2005 181 062
- E. NÜRNBERG ET AL.: "Poloxamere-was ist das?" DEUTSCHE APOTHEKER ZEITUNG, Bd. 129, Nr. 41, 12. Oktober 1989 (1989-10-12), Seiten 2183-2187, XP000071948 Stuttgart, DE
- DATABASE WPI Week 198128 Derwent Publications Ltd., London, GB; AN 1981-50707D XP002449469 & JP 56 060565 A (DAINICHISEIKA COLOR & CHEM MFG) 25. Mai 1981 (1981-05-25)

## Beschreibung

Die vorliegende Erfindung betrifft ein grundsätzliches Verfahren, um verschiedene Stoffe zu solubilisieren, zu dispergieren, zu stabilisieren und bedarfsweise zu maskieren. Bei diesen Stoffen handelt es sich zum Beispiel um lipophile und harzartige Wirkstoffe in hydrophilem Milieu wie auch um hydrophile Stoffe in lipophilem Milieu, aber auch um in wässrigen Systemen zu maskierende hydrophile Substanzen wie etwa solche, die bitter schmecken oder nach Fisch riechen. Unter dem Maskieren wird verstanden, dass Gerüche und Geschmacke von Stoffen unterdrückt werden. Und schliesslich geht es auch um feste Teilchen wie zum Beispiel Pollen und Hautschuppen in wässrigem Milieu. Mit dem Solubilisieren und Dispergieren solcher Stoffe wird, wenn es sich dabei um Wirkstoffe handelt, gleichzeitig auch eine Stabilisierung derselben in hydrophilem Milieu erreicht. Die Erfindung betrifft desweitern allerlei Halbfabrikate, Konzentrate und gebrauchsfertige Produkte als Erzeugnisse, die nach diesem Verfahren hergestellt sind. Namentlich ermöglicht es die Erfindung, Wirkstoffe, die sich für das Pflanzenwachstum als vorteilhaft erweisen, in einfach applizierbarer Form bereitzustellen. Sie können hierzu staubfrei zerkleinert, solubilisiert, dispergiert und bedarfsweise stabilisiert und dann appliziert werden, so dass sie den Pflanzen zur Aufnahme zur Verfügung stehen und von diesen auch tatsächlich aufgenommen werden. Dieses Solubilisierungsverfahren steigert die Effizienz der Anwendung von Pflanzenwirkstoffen enorm, und zudem bringt die staubfreie Durchführung eine riesige Kosteneinsparung, denn die Herstellumgebung muss nicht mehr sehr teuer vor der enormen Toxizität der Wirkstoffstäube (Herbizide, Fungizide, Akarizide, Pestizide) geschützt werden. Mit dem Solubilisieren und Dispergieren solcher Wirkstoffe für Pflanzen wird auch die Stabilisierung dieser Wirkstoffe in hydrophilem Milieu erreicht. Mit dem Stabilisieren ist nicht nur die Stabilität des gebrauchsfertigen Solubilisats gemeint, sondern auch dessen Stabilität auf dem Weg durch die Erde zur Wurzel. Die Erfindung erstreckt sich demnach auch auf die nach dem Verfahren entwickelten Wirkstoff-Formulierungen für Pflanzen als Erzeugnisse. Schliesslich umfasst die Erfindung auch die Verwendung derartiger solubilisierter Erzeugnisse für verschiedene kommerzielle Zwecke, soweit diese nicht die therapeutische Behandlung des menschlichen öder tierischen Körpers betreffen.

Man sucht generell Hilfsstoffe, die Solubilisierungen und Dispergierungen auslösen, zulassen, unterstützen oder erleichtern. Ideal ist es, wenn solche Hilfsstoffe bereits in Arzneimittel- oder Futtermittel-Listen enthalten sind, weil sie dann in keiner Art und Weise bedenklich sind und keine Einwände oder Vorbehalte seitens von Gesundheitsbehörden, Konsumentenorganisationen oder anderen Interessengemeinschaften zu befürchten sind. Doch nicht nur die reine Solubilisierung und Dispergierung eines Stoffes, namentlich eines Wirkstoffes, ist ein Thema, denn oftmals gilt es auch, einen solubilisierten Wirkstoff zu stabilisieren, damit er möglichst lange und vollständig seine Wirkung entfalten kann und seine Resorption verbessert wird.

Es gibt einige Wirkstoffe, deren Wirksamkeit in Fachkreisen bekannt ist, deren Applikation jedoch Schwierigkeiten bereitet, weil ihre Solubilisierung und namentlich auch ihre Stabilisierung Probleme bereitet. Genannt sei als Beispiel eines Wirkstoffes das Coenzym Q10. Studien aus den U.S.A. zeigten auf, dass dieser Wirkstoff eine wichtige Rolle bei der Bekämpfung von Parkinson und Altzheimer spielen könnte, aber auch positive Wirkungen zeigt bei Krebs, bei AIDS und anderen Infektionskrankheiten. In der Sportmedizin wurde bereits erkannt, dass Coenzym Q10 die Regeneration nach Verletzungen beschleunigt. Nun ist aber Coenzym Q10 ein fettlösliches Pulver und somit nicht wasserlöslich. Wirkstoffe aber, die nicht wasserlöslich sind, weisen oft eine schlechte Bioverfügbarkeit auf, das heisst sie können ihre Wirkung in einem Organismus nur wenig effizient und wenig zielgerichtet entfalten. Gerade bei besonders teueren Wirkstoffen ist es sehr ärgerlich, dass der grösste Teil der verabreichten Menge vom Organismus gar nicht genutzt werden kann, sondern wieder ausgeschwemmt oder ausgeschieden wird. Und somit erreicht ein so teurer Wirkstoff wie Coenzym Q10 in der praktischen Anwendung eine Bioverfügbarkeit von wohl bloss um die 10%.

Nach Bekanntwerden von Studienergebnissen, wonach Coenzym Q10 wie erwähnt für die Bekämpfung von Parkinson, Altzheimer, Krebs und AIDS bedeutsam ist, hat sich dessen Marktpreis in Kürze verdoppelt und er liegt derzeit bei ca. CHF 2'000.- pro kg. Es ist klar, dass die Steigerung der Bioverfügbarkeit eine grosse technische aber auch eine grosse ökonomische Verbesserung bedeuten würde. Wenn die Bioverfügbarkeit nur schon verdoppelt werden könnte, so könnten mit der gleichen Menge verfügbaren Wirkstoffes doppelt so viele Menschen behandelt werden.

Die Möglichkeit, einen fettlöslichen Wirkstoff dieser oder ähnlicher Qualität zu solubilisieren und gleichzeitig zu stabilisieren würde ungeahnte Perspektiven eröffnen. So könnten derartig behandelte Wirkstoffe oder Medikamente nicht mehr bloss als Kapseln oral verabreicht werden, sondern man könnte die Wirkstoffe einem Getränk beimischen, zum Beispiel einem Sportlergetränk. Das Verfügbarmachen von gezielten, bisher fettlöslichen Wirkstoffen in Getränken eröffnete, dank der Tatsache, dass diese darin löslich wären und ausser dem stabil blieben, ganz neue Geschäftsfelder für die ganze Lifestyle-Branche. Desweitern würde der im wässrigen Milieu suspendierte Wirkstoff nicht zu über 80% an der PET-Flascheninnenwand hängen bleiben, sondern die Flasche vollständig mit dem wässrigen Vehikel verlassen und somit dem Körper des Trinkenden zugeführt.

Ein weiterer wichtiger Wirkstoff, dessen Applikation sich schwierig gestaltet, ist Insulin. Insulin ist ein heikles Peptid mit vielen Aminosäuren, und es weist eine besonders empfindliche räumliche Struktur auf. Um eine angemessene Bioverfügbarkeit zu erzielen, muss es gespritzt werden. Das ist für die Betroffenen unangenehm und jedes Mal ein umständliches Prozedere. Es wäre hochwillkommen und würde einen grossen Durchbruch bedeuten, wenn man Insulin oral verfügbar machen könnte. In der westlichen Welt leiden bald einmal ein Fünftel aller Menschen an einer Form von mehr oder weniger starker Diabetes, und der Anteil an übergewichtigen Menschen nimmt weiter zu, womit auch die Anzahl der Diabetiker Typ II zunehmen werden. Die Bedeutung von Insulin wird allein schon deshalb steigen und eine bessere Form der Verabreichung wäre sehr gefragt. Insulin ist ein Hormon und deshalb definitionsgemäss eine Substanz, die in kleinen Mengen wichtige Funktionen unseres Körpers steuert. Stark wirksame Steuersubstanzen müssen bei ihrem Einsatz tief dosiert werden. Oft werden auch die Hilfsstoffe der verwendungsfähigen Lösung möglichst tief dosiert. Auf der oralen Route wird das angewendete Solubilisat eine weitere Verdünnung erfahren (durch Speichel, Magen- und Verdauungssaft), so dass die kritische MizellKonzentration (cmc) der beim Solubilisat eingesetzten Tenside erfahrungsgemäss deutlich unterschritten wird. Dadurch bricht das Solubilisat, wonach das Insulin nicht mehr vor Enzymen und pH-Wechseln geschützt und deshalb inaktiviert ist. Nicht zuletzt daran scheiterte bisher eine orale Verabreichung von Insulin.

Aus der WO 01/19329 A (THE PROCTER & GAMBLE COMPANY) 22. MÄRZ 2001 ist als nächstliegender Stand der Technik ein Solubilisierungsverfahren bekanntgeworden, bei dem man mit Poloxamer und Propylenglykol eine Schmelze herstellt, in welche dann ein Alkohol-Premix eines in Wasser schlecht löslichen Wirkstoffes eingearbeitet wird und dann das Ganze final mit Wasser homogenisiert wird. Bei einer solchen Schmelze verhalten sich die Mizellen wie nach Lehrbuch: Sie fallen unterhalb der kritischen MizellKonzentration cmc auseinander und der lipophile Wirkstoff fällt aus. Solchermassen solubiliiserte Wirkstoffe sind ausserdem nicht in jedem Verhältnis miteinander mischbar, ohne dass die Kombinationen aus Einzelsolubilisaten eintrüben oder sich anderswie physikalisch-chemisch verändern. Es wurde in der Literatur noch nie ein Solubilisierungssystem beschrieben, welches viele verschiedene Wirkstoffe mizelliert und dessen Mizellen auch weit unter der cmc stabil bleiben, sodass also keine Ausfällung, kein Aufrahmen und keine zunehmende Opaleszenz bei starker Verdünnung stattfindet.

Neben medizinisch wirksamen Stoffen gibt es eine Reihe nichtmedizinischer Stoffe, die bisher nicht solubilisiert oder dispergiert werden konnten. Bei diesen Wirkstoffen handelt es sich zum Beispiel um lipophile oder harzartige Wirkstoffe in hydrophilem Milieu wie auch um hydrophile Stoffe in lipophilem und hydrophilem Milieu, und schliesslich auch um feste Teilchen in wässrigem Milieu wie etwa eine Suspension. In Versuchen wurden zum Beispiel Pflanzen-Wirkstoff-Partikel in ein höherprozentiges Solubilisat gemischt und dieses wurde dann nass gemahlen. Die schliesslich aufgeschwemmte Suspension wurde in Hammer- oder Emulsionsmühlen weiter gemahlen, bis die Pulverkorngrösse in der Suspension auf etwa 2 bis 5 Mikrometer reduziert war. Wenn dann eine solche Lösung zur Applikation, also zur Aufnahme durch eine Pflanze, in das Erdreich im Bereich ihres Wurzelwerks gegeben wurde, und man Wasser nachgoss, so nahm die Pflanze mit ihrem Wurzelwerk zur Überraschung im Wesentlichen bloss das Wasser und die Emulgatoren mit ihren Wurzeln auf, nicht aber wie erhofft die Pflanzenwirkstoffe selbst!

Ideal ist es, wenn die bereitzustellenden Pflanzenwirkstoffe bereits zugelassen sind und keine Einwände oder Vorbehalte seitens von Behörden, Konsumentenorganisationen oder anderen Interessengemeinschaften zu befürchten sind. Doch nicht nur die reine Solubilisierung und Dispergierung eines Pflanzenwirkstoffes ist ein Thema, denn es gilt auch, ihn der Pflanze tatsächlich und effizient verfügbar zu machen, sodass er von derselben über ihr Wurzelwerk auch in der Tat aufgenommen wird und er die Wirkung in der Pflanze bis zu seinem Abbau entfalten kann.

Es gibt einige Pflanzenwirkstoffe, deren Wirksamkeit in Fachkreisen bekannt ist, deren Applikation jedoch Schwierigkeiten bereitet, weil ihre Solubilisierung sowie insbesondere ihre Aufnahme durch die Pflanze Probleme bereitet. Gerade bei besonders teuren oder stark wirksamen Pflanzenwirkstoffen ist es sehr ärgerlich, dass der grösste Teil der verabreichten Menge von der Pflanze gar nicht genutzt werden kann, sondern im Boden liegen bleibt und ihn kontaminiert. Die Möglichkeit, einen fettlöslichen Pflanzenwirkstoff zu solubilisieren und gleichzeitig zu stabilisieren, würde ungeahnte Perspektiven eröffnen. So könnten derartig behandelte Pflanzenwirkstoffe intakt von den Pflanzen aufgenommen werden.

Es ist daher die erste Aufgabe der vorliegenden Erfindung, ein grundsätzliches Verfahren anzugeben, mittels dessen sich Stoffe solubilisieren, dispergieren und stabilisieren lassen. Das Verfahren soll zuverlässig, einfach und kostengünstig umsetzbar sein und für verschiedenste Stoffe einsetzbar sein.

Eine zweite Aufgabe der Erfindung ist es, eine Anzahl von Stoffen durch Anwendung dieses Verfahrens zu anwendbaren Erzeugnissen zu entwickeln und anzugeben.

Eine dritte Aufgabe der Erfindung ist es, Verwendungen einiger dieser verfahrensgemäss hergestellten solubilisierten Erzeugnisse für spezifische Zwecke anzugeben.

Die erste Aufgabe wird gelöst von einem Verfahren zum Solubilisieren, Dispergieren und Stabilisieren von Stoffen, das sich dadurch auszeichnet, dass einerseits ein Poloxamer und andrerseits ein Harz und/oder ein Tocopherol zu einer kombinierten Schmelze aufgeschmolzen wird und der zu behandelnde Stoff innig in dieser Schmelze dispergiert oder gelöst wird.

Eine zweite Aufgabe wird gelöst von einem Erzeugnis, bestehend aus einer Schmelze oder einem transparenten Gel auf der Basis von wenigstens einem Poloxamer in Kombination mit einem natürlichen oder einem künstlich zusammengestellten Harz und/oder einem Tocopherol, sowie einem darin solubilisierten und stabilisierten Wirkstoff, in einer Konsistenz zwischen fest über halbfest, das heisst Aspik-artig bis hin zu flüssig.

Die dritte Aufgabe wird gelöst durch verschiedene Verwendungen von Erzeugnissen gemäss obenstehender Zusammensetzung, welche spezifische Stoffe in solubilisierter Form enthalten, für spezifische kommerzielle Zwecke gemäss den Verwendungsansprüchen.

Als besonders wichtig erweist sich, dass sich die Hilfsstoffe, also hier das Poloxamer, das Harz oder das Tocopherol und der zu behandelnde Wirkstoff sich auf molekularer Basis zusammenschliessen und sich dann, und nur dann, nach Zugabe von Wasser zur Verhinderung einer Aushärtung Mizellen bilden, die auch noch in einer 1000-fach kleineren Verdünnung als die kritische MizellKonzentration cmc als beladene Mizellen stabil bleiben. Tocopherol ist ein definierter Stoff, der in vier verschiedenen Stereo-Isomeren vorkommt, und in Abhängigkeit davon eine ,IN VIVO' -Vitamin-E-Wirkung hat, oder eben nicht hat. Um diese hochwichtige Stabilität der Mizellen auch bei sehr geringen Konzentrationen zu gewährleisten, ist es unabdingbar, dass die Schmelze aus einem Poloxamer in Kombination mit einem natürlichen oder einem künstlich zusammengestellten Harz und/oder einem Tocopherol hergestellt wird und der zu solubilisierende Wirkstoff innig darin dispergiert wird. Diese Schmelze wird danach mit Wasser von gleicher Temperatur überschichtet, wodurch sich ein Gel bildet, und sie wird hernach homogenisiert. Der sehr essentielle Vorteil dieses Solubilisierungsverfahren ist also die damit erreichte Stabilität der Mizellen selbst Zehnerpotenzen unter der cmc! Dieses Resultat ist nur und nur auf dem Weg zu erreichen, dass ein Poloxamer aufgeschmolzen wird und der einzusetzende Wirkstoff und ein natürliches oder ein künstlich zusammengestelltes Harz und/oder ein Tocopherol innig in dieser Schmelze dispergiert wird.

Wenn Poloxamer nicht mit Lösungsmitteln verunreinigt ist, wird es bei ca. 57°C bis 58°C flüssig. Man findet sodann, dass sich in einer Poloxamer-Schmelze sehr viele lipophile wie auch hydrophile Stoffe gut lösen. Die Beigabe von einem natürlichen oder einem künstlich zusammengestellten Harz, oder wahlweise zusätzlich oder anstelle des Harzes einem Tocopherol, und diese Art der Solubilisierung, indem man in einer Schmelze also diese Hilfsstoffe auf molekularer oder quasi-molekularer Basis mit den zu solubilisierenden Stoffen zusammenbringt, erweisen sich als Schlüssel zur Verbesserung des Beladungsgrades der so hergestellten Wirkstoff-Mizellen und als Schlüssel zur Erzielung einer Stabilität der so hergestellten Mizellen auch weit unter ihrer cmc. Man kann so nicht nur mehr Wirkstoff mit gleich viel Hilfsstoffen zu Mizellen umhüllen, sondern die erzeugten Mizellen bleiben auch weit unter der kritischen Mizellkonzentration absolut stabil. Weiter sind nur solubilisierte Wirkstoffe, die nach dieser Schmelz-Methode mit Harz und/oder Tocopherol hergestellt wurden, in jedem Verhältnis miteinander mischbar, ohne dass die Kombinationen aus Einzelsolubilisaten eintrüben oder sich anderswie physikalisch-chemisch verändern.

Ein kritischer Punkt im Verfahren stellt die Temperatur der Poloxamer-Schmelze dar. Es ist bekannt, dass namentlich Peptide sehr empflindlich auf erhöhe Temperaturen reagieren. In dem Temperaturbereich von 40°C bis 60°C werden manche Eiweisse denaturiert. Die Verfärbung des Eiweisses beim Braten eines Ei's in weiss bei 56° C zeugt von einer solchen Eiweiss-Strukturänderung, die jedermann schon mal beobachten konnte. Fieber von über 40°C ist nicht zuletzt wegen des einsetzenden Eiweiss-Wandels für den Patienten gefährlich. Die Schmelze-Temperatur von Poloxamer lässt sich nun durch die Zugabe geeigneter Lösungsmittel senken. Hierfür eignen sich zum Beispiel ungiftige Lösungsmittel wie Glyzerin, Propylen-Glykol, Polyethylen-Glykol 400 etc. Durch Zugabe derartiger Zusätze lässt sich die Schmelzetemperatur von Poloxamer soweit reduzieren, dass auch als thermolabil bekannte Wirkstoffe wie zum Beispiel Insulin oder ein sensibler Pflanzenwirkstoff solubilisiert und stabilisiert werden können.

Als weiterer, für gewisse Anwendungen optionaler und in diesem Fall wichtiger Verfahrensschritt erweist sich, dass die Poloxamer-Schmelze mit dem darin gelösten Wirkstoff für bestimmte Anwendungen sogleich mit einer genügend dicken Schicht Wasser von etwa gleicher Temperatur überdeckt wird. Durch diese Massnahme bildet sich unter dem Wasser von selbst sogleich ein transparenter Gel. Ohne eine solche Überdeckung mit gleichwarmem Wasser härtet die Schmelze aus und wird hart wie ein Kunststoff und ist in dieser Form nicht mehr direkt zu applizieren. Die Schmelze muss also zur Verhinderung einer Aushärtung noch in flüssigem Zustand mit etwa gleichwarmem Wasser überschüttet bzw. überschichtet werden. Mit kaltem Wasser funktioniert die Gelbildung ebenfalls, aber dann wird vor allem eine Dispergierung des Wirkstoffs entstehen. Nachdem Wasser von gleicher Temperatur dazugegeben wurde und dieses Wasser eine Überdeckung der Schmelze bildet - das Wasser schwimmt nämlich über der Schmelze auf - tritt spontan die Gel-Bildung ein und der Gel wächst rasch in dem Masse gegen die Wasseroberfläche nach oben, in dem die Schmelze Wasser aufnimmt. Diese von aussen beobachtbare Gelbildung wird unterstützt durch das rasche in Verbindung bringen von Wasser und Schmelze, etwa durch gezieltes Umrühren. Der Gel hat eine mizelläre Struktur mit einem Tröpfchendurchmesser von weniger als 80nm, sodass das Licht nicht daran gebrochen wird und der Gel daher klar durchsichtig ist, ja man sogar eine Zeitung durch ihn hindurch lesen kann, obwohl doch zum Beispiel etwa 5-10% davon fettlösliche Wirkstoffe sind, ca. 10-20% ein Poloxamer sind und 1-15% ein natürliches oder ein künstlich zusammengestelltes Harz und/oder anstelle davon ein Tocopherol sind. Diese Mizellen bleiben thermostabil, sodass selbst beim Kochen des Gels keine Trübung resultiert und auch durch die massenhafte Zugabe von Wasser die mizelläre Struktur nicht aufgebrochen wird. Die Konsistenz ist sirupartig oder dünner. Der Gel wird durch Rühren homogenisiert und durch Zugabe von Wasser oder Wasser-Lösungsmittel-Gemischen auf eine geeignete Viskosität verdünnt. Wenn man jedoch mit grossen Scherkräften homogenisiert, so ist das der Gelbildung abträglich. Dann wird der entstehende Gel nicht transparent, was bedeutet, dass neben der Solubilisierung auch eine Dispergierung stattfindet. Wird aber mit normalen Messern gerührt, etwa mit einer Stefan-Rührmaschine, welche eine senkrecht aus dem Behälterboden aufragende Drehachse mit senkrecht dazu angeordneten scharfen Messern aufweist, welche die Rührmasse immer wieder schneiden, oder mit einer Diosna-Rührmaschine, so ergibt sich sehr schnell ein optisch reiner, schöner und transparenter Gel mit wenig Luftblasen. In einer solchen Matrix schreitet eine Verkeimung wesentlich langsamer voran als in einer Flüssigkeit.

Das grundsätzliche Verfahrensprinzip beinhaltet breit formuliert, dass man die Wirkstoffe mit zwei obligatorischen Hilfsstoffen, nämlich einem Poloxamer einerseits und andrerseits einem Harz und/oder einem Tocopherol vermischt, und wenn eine extra tiefe Schmelztemperatur gewünscht oder nötig ist, zusätzlich fakultative Lösungsmittel wie Glyzerin, Propylen-Glykol, Polyethylen-Glykol 400 etc. zugegeben werden, und daraus eine Schmelze bei ca. 40°C bis 100°C erzeugt wird. In dieser werden alle zu solubilisierenden Komponenten in intensiven Oberflächenkontakt gebracht und anschliessend für bestimmte Anwendungen mit Wasser oder Kombinationen aus Wasser und fakultativen Lösungsmitteln überschichtet, wobei diese Überschichtung eine Temperatur von 1°C bis 100°C haben kann.

Durch die Solubilisierung und Stabilisierung des begehrten, die Zell-Mitochondrien stimulierenden Wirkstoffes **Coenzym Q10** entsteht bei der Überdeckung mit heissem Wasser ein dunkelroter transparenter Gel. Im Falle des Wirkstoffes Propolis hingegen fällt der transparente Gel dunkelgelb aus. In jedem Fall aber entsteht durch die Überdeckung mit heissem Wasser ein durchaus homogener und transparenter Gel. Sobald dieser Gel entstanden ist, kann das überschüssige Wasser abgegossen werden und der Gel kann aus dem Behälter entnommen werden. Er erweist sich als sehr robust und kann geknetet, gepresst, gezogen oder verdreht werden, ohne dass sich seine Konsistenz ändert. Je nach Art des durch dieses Verfahren mit Poloxamer und Harz bzw. Tocopherol solubilisierten Wirkstoffes entstehen verschiedene Farben im Gel. Durch den Einsatz weiterer Hilfsstoffe und durch Dosieren der verwendeten Wassermenge kann die Viskosität des Gels variiert werden. Je mehr Wasser eingerührt wird, umso flüssiger wird der Gel. Umgekehrt, wie spärlicher Wasser zugegeben wird, umso dickflüssiger wird der Gel, bis hin zu einer Konsistenz ähnlich wie Aspik bzw. Sulz. Diese Variabilität der Konsistenz eröffnet verschiedene Applikationsmöglichkeiten, etwa die Anwendung als wasserähnliches Konzentrat zum Zusetzen in Getränken, oder die Anwendung als dickflüssiger Gel zum Einsatz in Hautcremes bis hin zu streichfähigen Konsistenzen für Pflegemittel oder für Schmiermittel mit Fetten.

Dank dieses Verfahrens lässt sich die Bioverfügbarkeit eines Wirkstoffes wie etwa des Coenzyms Q10 bei oraler Verabreichung auf ca. 85% steigern, weil nun die Wirkstoffe auf molekularer Basis eingeschlossen sind und durch die Umschliessung mittels Poloxamer und Harz bzw. Tocopherol auch unter der cmc von Poloxamer stabil bleiben. Bedenkt man, dass im Jahr 2004 die weltweit abgesetzte Menge Coenzym Q10 bei ca. 100 Tonnen lag, und der kg-Preis bei CHF 2'000.- liegt, so kann auch die ökonomische Tragweise einer wesentlichen Steigerung der Bioverfügbarkeit ermessen werden.

Ein bedeutsamer Punkt im Zusammenhang mit dem offenbarten Verfahren ist, dass Poloxamer, Harz und Tocopherol in den Standard-Nachschlagewerken für die Apotheker und Pharmaindustrien aufgeführt sind, nämlich in den internationalen Pharmacopoeas. Nicht nur bekannte Wirkstoffe und Hilfstoffe sind in diesen Pharmacopoeas ausführlich beschrieben, sondern es ist diesen Werken auch zu entnehmen, wie Stoffe zu sein haben, damit man sie benützen darf. Deren Reinheit, deren Gehalte, Rückstände etc. werden angegeben. Ausserdem ist darin beschrieben, wie man mit diesen Hilfs- und Wirkstoffen umgeht etc. Es existiert unter anderem für die U.S.A. die US-Pharmacopea der FDA, in der Europäischen Union gilt die EU-Pharmacopoea, Grossbritannien hat eine eigene britische Pharmacopoea, weiter gibt es auch eine japanische Pharmacopoea. Dieser kommt sogar eine Vorreiterrolle zu, weil sie besonders streng ist. Auch Russland und China haben ihre eigenen Pharmacopoeas. Wenn ein Hilfsstoff Eingang in die Pharmacopoea findet, dann ist er im Rahmen der darin beschriebenen Auflagen überall einsetzbar.

In den verschiedenen Pharamcopoeas haben Poloxamer, diverse Harze und Tocopherol eine eigene Monografie, d.h. einen kompletten Substanzenbeschrieb. Die Poloxamere sind völlig inert. Harze sind systemisch selten angewendet und nicht stark wirksam und Tocopherole (Vit. E) oft angewendet und schwach wirksam. In keinem Land stösst man mit diesen Stoffen auf Widerstände, und in vielen Kosmetikas, Pharma- und Tierernährungsergänzungs-Produkten werden diese Hilfsstoffe bereits angewendet.

Im Folgenden werden einige Wirkstoffe erwähnt, für deren Solubilisierung und Stabilisierung sich das Verfahren eignet. **Vitamin C** ist ein sehr hilfreicher Wirkstoff und als solcher wasserlöslich. Vitamin C ist aber nicht besonders stabil gegen Licht, Luft und in neutralem pH-Bereich. Wenn Vitamin C in Wasser aufgelöst ist, dann wird es in 2 bis 3 Tragen hellgelb, dann gelb und schliesslich braunrot, was ein ganz deutliches Zeichen dafür ist, dass es sich strukturell verändert und deshalb auch seine Wirkung verliert. Deshalb nutzt man Vitamin C in verschiedenen Produkten zurückhaltend oder gar nicht, obwohl seine Wirkung anerkannt ist und erwünscht wäre, etwa in der Kosmetik und der Ernährungsergänzung. Mittels des hier offenbarten Verfahrens lässt sich Vitamin C stabilisieren.

Ein weiterer bedeutsamer Wirkstoff ist auch **Insulin.** Insulin ist an sich wasserlöslich, aber in Bezug auf seine Stabilität sehr heikel. Die meisten Applikationen neben dem Spritzen führen rasch zur Inaktivierung des Insulins. Wenn man es zum Beispiel oral verabreicht und somit durch den Magen-Darmtrakt schickt, trifft es in der Folge auf Salzsäuren und Pepsin im Magen, und danach auf den Dünndarmsaft mit Cholesterin und Gallensäure. Durch diese Verdauungssäfte wird die Wirkung des Insulins aufgehoben. Mittels des Dispergierungs- und Stabilisierungsverfahrens wie oben beschrieben lässt sich aber die Bioverfügbarkeit von Insulin wesentlich steigern, sodass eine orale Verabreichung interessant wird.

Es gibt weiter eine Reihe von Wirkstoffen, die herkömmlich durch einen **Nasenspray** in die Nase gespritzt werden. Manche solche Sprays enthalten Hilfsstoffe, welche - besonders wenn der Spray über längere Zeiträume angewendet wird - Allergien in den Nasenschleimhäuten, in den Atemwegen oder gar in der Lunge auslösen können. Solubilisiert und stabilisiert man diese Wirkstoffe mit Poloxamer und Tocopherol wie oben beschrieben, so reagieren die Allergiker nicht mehr auf ihr **solubilisiertes Allergen.** Poloxamer und Tocopherol rufen selbst keine Allergien hervor, weder im Einsatz als Nasenspray noch in einer Trinklösung, und sie isolieren Allergene äusserst effektiv.

Stets erweist es sich als kardinal, dass aus dem Poloxamer und Harz bzw. Tocopherol eine Schmelze erzeugt wird, die dann für bestimmte Anwendungen mit Wasser gleicher Temperatur überschichtet wird. Ein spezielles Poloxamer, nämlich Lutrol F68, erzeugt im Verfahren eine geringere Viskosität und kann daher zur Solubilisierung und Stabilisierung von anschliessend parenteral zu applizierenden Wirkstoffen eingesetzt werden, sei es über Infusionen oder Injektionen. Dabei ist bei fachgerechter Fertigung die Gefahr einer Fettembolie durch den lipophilen Wirkstoff klein, weil er im Blut nicht ausfällt.

Kontaktlinsen, deren regelmässige Reinigung sehr wichtig ist, können dank des Einsatzes einer **Reinigungslösung** auf der Basis dieses Solubilisierungs-Verfahrens gesäubert werden, ohne anschliessend Augenbrennen auszulösen. Solche Kontaktlinsen sind heikel zum Reinigen. Neu können sie mit Hilfe von Lösungen nach der hier vorgestellten Technologie elegant und gründlich gereinigt werden, sogar ohne dass sie vom Auge entfernt werden müssen.

Wird Poloxamer, Harz bzw. Tocopherol verfahrensgemäss eingesetzt, so können lipophile und harzartige Wirkstoffe in hydrophilem Milieu gelöst werden wie auch hydrophile Stoffe in lipophilem Milieu. Es können auch feste Teilchen in wässrigem Milieu solubilisiert und dispergiert werden. Eine Anwendung liegt zum Beispiel darin, Pollen in wässrigem Milieu zu isolieren und abzutransportieren. Es lässt sich ein Nasenspray herstellen, welcher in die Nase eingedrungene Pollen, die dort die Schleimhäute reizen, dispergiert, sodass zum Beispiel ein Heuschnupfen unterbunden wird. Eingesetzt in einem **Shampoo** können Hautschuppen bekämpft werden, indem diese in ähnlicher Weise in wässrigem Milieu dispergiert werden und sich dann leicht und ohne nennenswerte mechanische Einwirkung von der Haut und insbesondere von der Kopfhaut lösen lassen. Es werden dadurch also auch kosmetische und dermatologische Anwendungen möglich, egal ob an Mensch oder Tier.

Desweiteren können **Getränke und Sirupe** zur Nahrungsergänzung von Mensch und Tier, sowie reine Getränke für Mensch und Tier hergestellt werden, die wahlweise auch mit Sauerstoff angereichert sein können und aus denen die "Wirkstoffe" gut resorbiert werden. Das Verfahren ist wie schon beschrieben hoch geeignet, um auch lokal zu verabreichende Arzneimittel für Mensch und Tier bereitzustellen, sowie parenteral zu verabreichende Arzneimittel und Lösungen für Mensch und Tier, die Sauerstoff angereichert sind. 5°C bis 10°C kaltes Tiefen-Quellwasser enthält etwa pro Liter 12mg bis 18mg Sauerstoff, und wenn das Wasser erwärmt wird, sinkt dessen Sauerstoffgehalt rasch ab. Durch verfahrensgemäss hergestellte Mizellen erhöht sich die innere Oberfläche, an der man Sauerstoff anlagern kann. Eine 1 %-ige, wässrige, unbeladene Mizell-Lösung hält dann über 100mg Medizinal-Sauerstoff pro Liter Wasser bei 18°C in einem zu 2/3 gefüllten, deckellosem 10-Liter-Eimer über 5 Tage stabil fest! Schliesslich eröffnen sich auch in technischen Anwendungen eine Vielzahl von Anwendungen der nach dem Verfahren erzeugten Stoffe. Es lassen sich Gegenstände von Fettrückständen befreien. Andererseits können durch das Solubilisieren von Fetten auch Schmierstoffe und fetthaltige Pflegemittel hergestellt werden.

Für folgende Stoffe und Anwendungen eignet sich das vorgestellte Verfahren insbesondere: Grundsätzlich für die Solubilisierung lipophiler und hydrophiler Stoffe für Emährungsergänzungen, für Kosmetika und Dermatologika, wie Coenzym Q10, Vitamin C, Vitamin E, Betacarotin, Vitamin A, Vitamin D3, Lutein, Lycopene, Folsäure, Vitamin B12, Ω-3- und Ω-6-Fettsäuren, für harzartige Stoffe gegen Infektionen, für die Konservierung von Stoffen, zum Herstellen von Stoffen für die Wundversorgung, zum Beispiel auf der Basis von Propolis, Selendioxid, Teeren und Steinölen. Desweiteren ermöglicht es das Verfahren, Peptide oral bioverfügbar zu machen, wie etwa Insulin (Antidiabetika), Ciclosporin etc., sowie Planzenextrakte aus Mariendistel, Passionsblume und Pestwurzel etc. und deren Leitstoffe (wie Silymarin, Chrysin etc.) zu solubilisieren. Ganz generell lassen sich verfahrensgemäss problemlos Polyphenylverbindungen solubilisieren , die im Solubilisat immer einen Wirkstoffgehalt von 2-5% erreichen.

Folgende quantitativen Zusammensetzungen der Gele aus den Poloxamer-Harz- (bzw. Tocopherol)-Schmelzen sind typisch: Wirkstoff/e 1% bis 10%, Total-Poloxamer-Gehalt 10% bis 20%, Harz bzw. Tocopherol 1 % bis 20% und der Rest Wasser auf 100% Gel-Gewicht. Gele, die nach den beschriebenen Verfahren via eine Schmelze hergestellt werden, zeichnen sich durch ihre Transparenz aus, die auch bei noch so grossen anschliessenden Verdünnungen mit Wasser oder Wasser-Lösungsmittel-Gemischen erhalten bleibt. Nur durch die Herstellung über Schmelzen mit Poloxamer/en und Harz bzw. Tocopherol, und Wirkstoff/en und allenfalls fakultativen Lösungsmitteln können so hohe Wirkstoffmengen zu einem völlig transparenten Gel solubilisiert werden. Durch die geeignete Zugabe von Lösungsmitteln kann die Viskosität des Gels gesenkt und/oder die Schmelze bei tieferen Temperaturen gefertigt werden. Bei diesem Herstellverfahren lassen die überschichteten Hilfsstoff-Schmelzen eine Vielzahl von Solubilisierungen zu: So kann etwa der Wirkstoffgehalt solubilisierter, hydrophiler Ascorbinsäure in Öl gut 10% betragen, ebenso kann lipophilen Coenzym Q10 in Wasser mit 2%-6% genauso solubilisiert werden wie harzartiges Propolis.

Im Falle des Wirkstoffes Insulin wird nicht nur eine homogene, stabile und konzentrierte Dispergierung erreicht, sondern auch die orale Bioverfügbarkeit dieses Wirkstoffes massiv verbessert. Da die Poloxamere in allen wichtigen Arzneibüchern verankert und zum Beispiel in der Schweiz sogar als Zusatzstoff der Futtermittelliste aufgeführt sind, sind sie unbedenklich für den oralen Gebrauch und in der Qualität des Poloxamers 188 sogar parenteral nutzbar. Nachfolgend werden einige detaillierte Beispiel angegeben, wie das Verfahren umgesetzt werden kann.

### Beispiel 1

Stabilisierung von Vitamin C und ACC in einem ACC-, Vitamin C- und Vitamin E-haltigen Shampoo:

| | | |
|---|---|---|
| A | Wasser | 74.11 % |
| B | Sodium Laureth Sulfate | 11.67 % |
| C | Cocamidopropyl Betain | 2.00 % |
| D | Cocamide DEA | 1.70 % |
| E | Disodium Laureth Sulfosuccinate | 1.32 % |
| F | Parfum | 1.00 % |
| *G* | *Poloxamer 407* | *1.40 %* |
| *H* | *Poloxamer 188* | *0.60 %* |
| I | Ascorbinsäure | 1.00 % |
| J | Acetyl Cysteine | 1.00% |
| K | Alfa-Tocopherole | 0.50 % |
| L | Sodium Lauryl Sulfate | 0.80 % |
| M | Phenoxyethanol | 0.50 % |
| N | Imidazolidinyl Urea | 0.20 % |
| O | PEG-120 Methyl Glucose Dioleate | 0.10 % |
| P | Tetrasodium EDTA | 0.10 % |

Das Herstellungsverfahren läuft wie folgt ab:
- G, H und K auf 60°C bis zur Schmelze erwärmen,
- I und J mischen und unter Rühren in der Schmelze dispergieren,
- Mit einem Viertel von A (60°C warm) überschichten und die Gelbildung abwarten → Gel,
- Drei Viertel von A vorlegen und nacheinander unter Rühren B, C, D, E, F, L, M, N, O, P einwägen → transparente Lösung,
- Gel vorlegen und unter Rühren die Lösung zugeben.

### Beispiel 2

Solubilisierungsbeispiel für Coenzym Q10 in einem Vitamin C- und Vitamin E-haltigen Gel:

| | |
|---|---|
| Aqua | 71.43% |
| *Poloxamer 188* | *8.93%* |
| *Poloxamere 407* | *8.93%* |
| Alfa-Tocopherol | 5.00% |
| Coenzym Q10 | 2.14% |
| Asccorbinsäure | 3.57% |

### Beispiel 3

Solubilisierungsbeispiel für einen Propolis-haltigen Gel:

| | | | |
|---|---|---|---|
| Aqua | 70% | | |
| *Poloxamer 188* | 18% | | |
| Propolis | 12% | | |

Zur Präzisierung und Klarstellung werden hier einige Definitionen und Erklärungen gegeben:
- Unter Tensiden versteht man Verbindungen, deren Moleküle einen wasserliebenden (hydrophilen) und einen fettliebenden (lipophilen) Teil enthalten.
- In Folge dieser Tatsache reichem sich Tenside in der Grenzfläche der Wasserphase an, d.h. sie sind grenzflächenaktiv. Das tun sie unabhängig davon, ob an die Wasserphase eine gasförmige, eine flüssige oder eine feste Phase stösst.
- Darüberhinaus bilden sich im Inneren der Lösung beim Überschreiten einer bestimmten Tensidkonzentration grössere Molekülverbände aus Tensiden, die mit den Einzelmolekülen in einem Gleichgewicht stehen. Die Molekülverbände können von unterschiedlicher Grösse und Gestalt sein, haben aber im einfachsten Fall Kugelgestalt.
- Beim Überschreiten einer bestimmten, für das jeweilige Tensid charakteristischen Konzentration lagern sich Tensidmoleküle so zusammen, dass ein Gebilde entsteht, dessen Inneres aus lipophilen Resten besteht, und an dessen Oberfläche die hydrophilen Gruppen sitzen, die den Kontakt zum Wasser halten und so auch die Löslichkeit des Gebildes in Wasser bestimmen.
- Die Aggregate heissen Mizellen und diese Molekülverbände können sich auch wieder auflösen, wenn man das System solange mit Wasser verdünnt bis die Konzentration der Tenside unter die charakteristische Grösse fällt, die man "kritische Mizellbildungskonzentration" nennt, abgekürzt "cmc".
- Die "cmc" eines Tensids ist um so grösser, je weniger lipophil der unpolare Anteil des Tensid-Moleküls ist.
- Das Innere einer Mizelle befindet sich im flüssigen Zustand.
- Unterhalb der "cmc" liegen in Lösungen in den meisten Fällen nur Mono-Tenside vor. Oberhalb der "cmc" bleibt die Anzahl der Aggregate fast immer konstant. Das ganze zusätzliche Tensidmaterial geht oberhalb der "cmc" nur noch in die mizellare Form, so dass die Mizellbildung als Bildung einer neuen Phase betrachtet werden kann, bei der allerdings die Aggregationszahl nicht ins Unendliche wächst.
- Die Konzentration des gelösten Mono-Tensids kann also nicht über die "cmc" • hinaus gesteigert werden.
- Die Temperatur, bei der die Auflösung des Tensids infolge einsetzender Mizellbildung erfolgt, heisst der Krafft-Punkt des Tensids. Da dieser KrafftPunkt eine ziemlich scharf definierte Temperatur darstellt, hat es den Anschein, als würde das ungelöste Tensid beim Erreichen des Krafft-Punktes schmelzen. Der Krafft-Punkt wird daher oft mit dem Schmelzpunkt verglichen.
- Nichtionische Tenside, die in Wasser eine klare Lösung bilden, zeigen im Gegensatz zu anderen Tensiden ein besonderes Verhalten. Bei Temperaturerhöhung wird die Lösung beim Überschreiten einer bestimmten, relativ scharf definierten Temperatur, die für das jeweilige Tensid charakteristisch ist, trüb und es erfolgt die Auftrennung der Lösung in zwei flüssige Phasen. Diese Temperatur wird Trübungspunkt genannt.
- Der Grund für das Auftreten dieser Erscheinung hängt mit der Hydratation der hydrophilen nichtionischen Gruppen zusammen. Bei steigender Temperatur erfolgt eine teilweise Dehydratisierung und dadurch die Bildung einer neuen Phase. Deshalb ist der Trübungspunkt fast unabhängig von der Gesamtkonzentration an Tensid. Diese Trübung ist jedoch bei Abkühlung reversibel. Der Trübungspunkt kann durch Additive beeinflusst werden: Es können Additive in die Mizellen eingelagert werden und deren Eigenschaften verändern oder sie können die Eigenschaften des Wassers, also der Umgebung der Mizellen, verändern. Dieser letztere Mechanismus trifft besonders auf den Zusatz von Elektrolyten zu, die generell den Trübungspunkt mit steigender Konzentration zu tieferen Temperaturen verschieben.
- Die Kugelgestalt der Mizellen kommt zustande, weil die hydrophilen Kopfgruppen infolge elektrostatischer Abstossung möglichst weit von einander entfernt sein wollen und gleichzeitig möglichst alle Kontakt mit den umgebenden Wassermolekülen haben möchten. Die lipophilen Molekülteile im Inneren der Mizellen sind nicht in Kontakt mit dem Wasser; die Grösse der Mizellen ist durch den Platzbedarf der lipophilen Reste gegeben.

### Für Pluronic gelten folgende Daten:

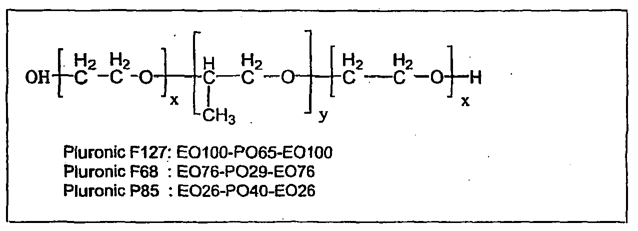

| Polymer | Molekulargewicht | HLB-Wert | CMC (g/It) | Mizell Durchmesser |
|---|---|---|---|---|
| Pluronic P85 | 4600 | 26 | 1,058 | 1.5 nm |
| Pluronic F68 | 8400 | 29 | 1,344 | 1.3 nm |
| Pluronic F127 | 12600 | 22 | 0.869 | 3.3 nm |

(Z. Sezgin et al./ European Journal of Biopharmazeutics 64 [2006] 261-268)

Es folgen weitere klärende Erläuterungen:
- Bei kugelförmigen Mizellen kann der Radius nur bis zur Länge des lipophilen Molekülteils anwachsen. Die Zahl der Mono-Tenside pro Kugelmizelle (= Aggregationszahl) ist damit nach oben begrenzt und gegeben durch den Volumenbedarf eines lipophilen Molekülteils im Verhältnis zum Gesamtvolumen der Mizelle.
- Bei einer Konzentrationserhöhung des Tensids wird daher die Grösse der Mizellen konstant bleiben und deren Zahl zunehmen.
- Eine wichtige Eigenschaft der Mizellen ist ihre Fähigkeit, andere Moleküle zu solubilisieren. Da Mizellen praktisch kleine Kohlenwasserstofftröpfchen darstellen, sind sie in der Lage, lipophile Stoffe aufzulösen. Diese wasserunlöslichen Stoffe werden in das Innere der Mizelle eingebaut und sind nicht mehr in Kontakt mit dem Wasser. Da aber die Oberfläche der Mizellen hydrophil ist, schwimmt der Wirkstoff nun gelöst im Wasser. Dieser Vorgang heisst Solubilisation. Mizellen sind im thermodynamischen Sinn stabile Systeme. Die Konzentration der durch Tenside solubilisierten Teilchen liegt meist unterhalb von 5%. Die Phasengrenzfläche bewirkt eine Lichtstreuung (Tyndall-Effekt), das System erscheint oft etwas trübe.
- Solubilisation (= Einlagern von lipophilen Stoffen ins innere von Mizellen) lässt die Mizellen wachsen, so dass zusätzliche Tenside in die Mizelloberfläche eingelagert werden. Der Durchmesser der Mizellen wächst dabei bei Pluronic F68 von 1,3nm (unbeladen) und bei Pluronic F127 von 3,3nm (unbeladen) an aufwärts. Der Mizelldurchmesser liegt normalerweise unter 140nm. Dieses Quellen der Mizellen kann aber zu Teilchengrössen des Solubilisats von bis zu 500nm führen, wobei dann das Solubilisat nahezu transparent mit einem bläulichen Schimmer erscheint. Bei Teilchengrössen unter 140nm erscheint das Solubilisat nur transparent.
- Der HLB-Wert (Hydrophile-Lipophile-Balance) quantifiziert den hydrophilen und den lipophilen Anteil im Tensidmolekül. Dieser Wert ist dann ein Ausdruck für die Eigenschaften des Tensidmoleküls. Der HLB-Wert von Pluronic F68 ist 29, der von Pluronic F127 ist 22. Damit sind beide Tenside starke Lösungsvermittler.
- Zur klaren Solubilisierung von Parfümölen, ätherischen Ölen und öllöslichen Vitaminen werden Tenside mit einem HLB-Wert von 14-17 eingesetzt. Diese Stoffe werden auch als Lösungsvermittler oder Solubilisatoren bezeichnet. Wegen ihrem hohen HLB-Wert neigen Lösungsvermittler im allgemeinen mehr oder weniger stark zum Schäumen, was technisch Probleme aufwirft.
   Beide Pluronics (F68 und F127) sind aber Schaumkiller.
- Vor allem Parfümöle, die Harze, Resinoide, Terpene, Ester oder Ketone enthalten, sind sehr schlecht wasserlöslich und müssen in wässrigen Systemen solubilisiert werden.
- Harze, Resinoide und ihre Ester sind umgekehrt auch sehr gute Lösungsmittel für ätherische Öle, öllösliche Vitamine, PolyphenylVerbindungen und andere lipophile Wirkstoffe. Gleichzeitig lassen sich Harze, Resinoide und ihre Ester besonders gut durch die Tenside Pluronic F127, Pluronic F68 und Pluronic P85 solubilisieren.

Abamectin ist eine Mischung von >=80% Abamectin B₁ₐ (M: 873,1; C₄₈H₇₂O₁₄) und <=20% Abamectin B_{1b} (M: 859,1; C₄₇H₇₀O₁₄). Es sind farblose bis schwach gelbe Kristalle mit einem Schmelzpunkt von 161,8-169,4 °C (unter Zersetzung) und einer Dichte bei 25°C von 1,18. Abamectin ist stabil gegen Hydrolyse in wässrigen Lösungen bei pH 5, 7 und 9 (bei 25°C). Mit etwa 2g Abamectin in 1000 Liter Wasser feinst verteilt lässt sich in etwa einer Stunde eine Fläche von einer Hektar (zwei Fussballfelder) besprühen. An einer Musterrezeptur für den Wirkstoff Abamectin, welcher ein sehr potentes Akarizid (gegen Milben) ist, kann gezeigt werden, dass über die gute Löslichkeit von Abamectin in einer Harzmischung hinaus, und über eine gute Solubilisierung der Harz-Abamectin-Lösung mittels Pluronic hinaus, eine einmalige, noch nie beobachtete Stabilität von der nach diesem Verfahren hergestellten Solubilisaten auch unter der für Pluronic charakteristischen "cmc" von 1,344g/lt (F68), bzw. 0,869g/lt (F127) und über der Löslichkeitsgrenze von 7-10 mcg/lt Abamectin in Wasser besteht (25°C).

Die wasserfreie Formulierung mit einer möglichst hohen Abamectin-Konzentration, die sich ohne Probleme mit Wasser in jedes Verhältnis bringen lässt und immer transparente Ergebnisse liefert, lautet wie folgt:

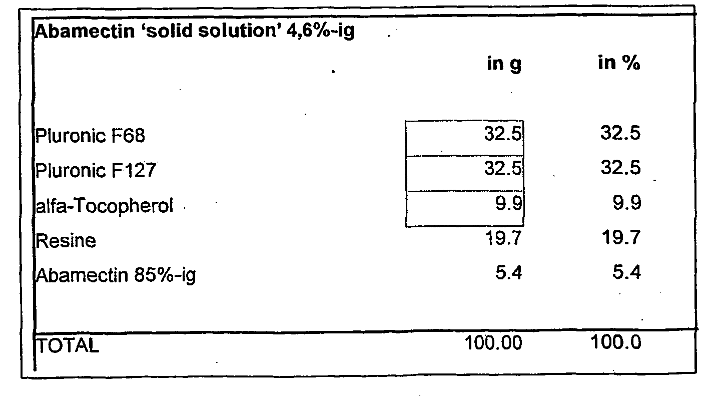

### Die Formulierung bei der "cmc" von Pluronic F127 (=0,869g/lt):

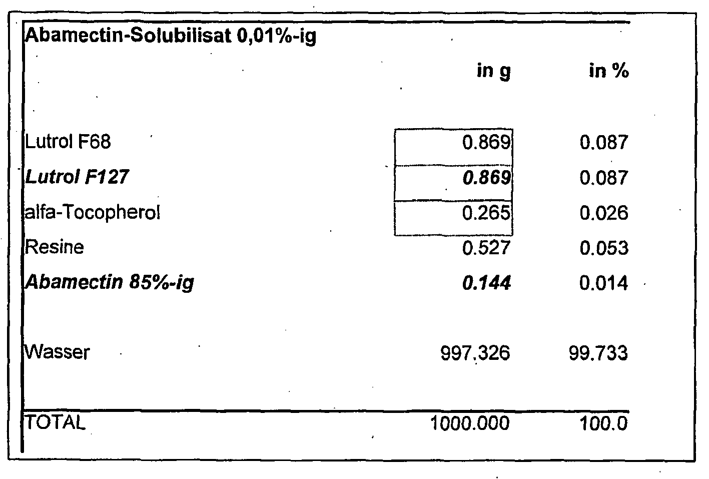

Mit 122'000mcg/lt liegt die Abamectin-Konzentration in Wasser bei der "cmc" von Pluronic F127 weit über der Löslichkeit von 7-10mcg Abamectin pro einem Liter Wasser. Trotzdem bleibt das Solubilisat bei weiterer Verdünnung mit Wasser stabil und transparent. Dieses Resultat ist mit keinem anderen bis jetzt bekannten Herstellungsprozess bei gleicher Formulierung erzeugbar. Die so hergestellten Mizellen bleiben auch unter der cmc stabil, wie wenn das eingesetzte Harz nicht nur Lösungsmittel für Abamectin ist und sich mit Pluronic gut solubilisieren lässt, sondern darüber hinaus gleichzeitig eine spezielle Bindung mit Pluronic eingeht und so die Mizellen auch bei extrem grosser Verdünnung mit Wasser stabil hält. Eine typische Einsatzkonzentration für Abamectin ist 2'000mcg/lt, also eine Konzentration, die oberhalb der Löslichkeitsgrenze für Abamectin in Wasser liegt und deshalb in Mizellen solubilisiert werden sollte.

### Eine Formulierung der Harzmischung (Resine):

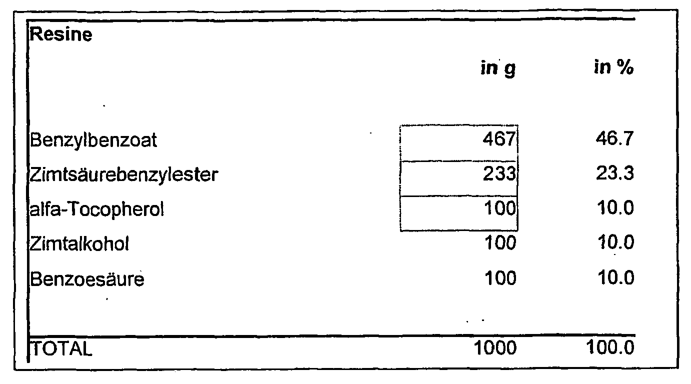

Folgende Komponenten werden eingesetzt: **Harze:**
- Benzylbenzoat (M: 212,2; C₁₄H₁₂O₂) ist eine farblose Flüssigkeit oder farblose Kristalle; praktisch unlöslich in Wasser. (verhindert Auskristallisieren).
- Zimtsäurebenzylester (M: 238,29; C₁₆H₁₄O₂; Dichte: 1,106) sind weisse, aromatisch riechende Kristalle; praktisch unlöslich in Wasser.
- oder wahlweise Benzylconiferylester. **Antioxidans:** Alfa-Tocopherol.
   **Lösungsmittel:** Zimtalkohol, Benzylalkohol, Ethyldiglykol, Dipropylenglykol, PEG 400, Benzoesäure.

Wenn man Abamectin-Solubilisate mit Wasser auf einen Wirkstoffgehalt zwischen etwa 2% und 0,5% einstellt, so erhält man gelförmige transparente Zubereitungen, die klingen, wenn man ein Glas mit diesem Gel auf eine harte Unterlage stösst. Dieses "Ringing" ist auch als Vibration spürbar. Derartige Ringing-Gele sind in der Kälte und Wärme ausserordentlich stabil. Diese stabile Struktur bewirkt auch, dass hydrolyseempfindliche Wirkstoffe durch die Einlagerung in das System selbst bei Aufbewahrung unter erhöhter Temperatur stabil bleiben.
Beispiele:

| **Abamectin-Solubilisat 1,6%-ig** | | |
|---|---|---|
| | **in g** | **in %** |
| | | |
| | | |
| Pluronic F68 | 130.17 | 13.0 |
| Pluronic F127 | 130.17 | 13.0 |
| alfa-Tocopherol | 34.2 | 3.4 |
| Resine | 68.48 | 6.8 |
| Abamectin 85%-ig | 18.6 | 1.9 |
| | | |
| Aqua | 618.38 | 61.8 |
| | | |
| | | |
| TOTAL | 1000.00 | 100.0 |

| **Abamectin-Solubilisat 0,86%-ig** | | |
|---|---|---|
| | **in g** | **in %** |
| | | |
| Pluronic F68 | 83.35 | 8.3 |
| Pluronic F127 | 83.35 | 8.3 |
| alfa-Tocopherol | 21.94 | 2.2 |
| Resine | 43.82 | 4.4 |
| Abamectin 85%-ig | 10.24 | 1.02 |
| | | |
| Aqua | 757.3 | 75.7 |
| | | |
| TOTAL | 1000.00 | 100.0 |

Wenn trockene Feststoffe in wasserdampfbeladener Luft ungeschützt gelagert werden, findet in Abhängigkeit vom Feuchtigkeitsgehalt der Luft und von hygroskopischen Eigenschaften des Feststoffes eine **Sorption,** d.h. eine Dampfaufnahme statt. Bei einem solchen Vorgang können entweder nur einige Molekülschichten auf die Feststoffoberfläche durch **Adsorption** gebunden werden oder in die Masse der Feststoffe eindringen, was als **Absorption** bezeichnet wird. Eine Desorption tritt hingegen dann ein, wenn feuchte Feststoffe in trockener Atmosphäre Wasserdampf abgeben.

Feste oder halbfeste Wirkstoffkonzentrate, die nach dem beschriebenen grundsätzlichen Verfahren durch Herstellen einer Wirkstoffschmelze oder eines Wirkstoff-Gels gewonnen werden, wobei sich diese Schmelzen und Gele dann als wasserdampfbeladene und somit hydrophile und/oder wasserlösliche Feststoffe mit grosser Oberfläche erweisen, oder die durch Sprüh- und/oder Gefriertrocknung vergrösserte Oberflächen aufweisen, bieten für die Resorbtion von Wirkstoffen im tierischen oder menschlichen Körper hervorragende Eigenschaften. Diese Eigenschaften sind unabhängig davon, ob die Wirkstoffkonzentrate final einer Desorbtion unterworfen werden oder nicht. Mit den oben beschriebenen Verfahren können daher feste Wirkstoffkonzentrate für den oralen Gebrauch hergestellt werden, die sich in Gelatine-Kapseln abfüllen lassen, als dosierte Pulver in Sachets abpacken oder zu Tabletten und Brausetabletten komprimieren lassen. Nach deren Zerfall und Auflösung in Wasser oder dem Magen/Darmsaft des Menschen oder Tieren liegen dann die Wirkstoffe in solubilisierter und/oder dispergierter Form vor, die sich schnell und vollständig resorbieren lassen. Desgleichen können so auch halbfeste Wirkstoffkonzentrate mit guten Resorbtionseigenschaften hergestellt werden, die sich anschliessend in Weichgelatinekapseln abfüllen lassen.

Als geeignete hydrophile oder wasserlösliche Feststoffe mit grossen Oberflächen oder durch Sprüh- und/oder Gefriertrocknung vergrösserten Oberflächen (spezifische Oberfläche > 0.01 m²/g BET-Methode) sind besonders geeignet:
- Exudate, wie z.B. Gummi arabicum, Traganth, Karayagummi, Ghattigummi,
- Samenmehle, wie z.B. Guargummi, Johannisbrotkernmehl, Tarakernmehl, Tamarindengummi,
- Gerüstsubstanzen, wie z.B. Lärchengummi, Pektin, Agar, Alginat, Carrageen, Furcellaran,
- Biosynthetische Hydrokolloide, wie z.B. Xanthan,
- Modifizierte Hydrokolloide, wie z.B. Propylenglykolalginate, amidiertes Pektin,
- Cellulose-Derivate, wie z.B. Methylcellulose, Methylethylcellulose, Methylhydroxyethylcellulose, Methylhydroxypropylcellulose, Hydroxypropylcellu-lose, Natrium-Carboxymethylcellulose,
- Siliziumoxide, wie z.B. Aerosil,
- Proteine, wie z.B. Gelatine, Magermilchpulver,
- Zucker, wie Laktose, Mannit, Xylit, Sorbit, Dextran.

Als weiterer Aspekt der Erfindung wird nun ihr Einsatz für die Applikation von Planzenwirkstoffen diskutiert. Zunächst wird das Verfahren zum Solubilisieren und Stabilisieren eines Pflanzenwirkstoffes näher vorgestellt. Als besonders wichtig hat sich nämlich erwiesen, dass sich der Wirk- und die Hilfsstoffe, also hier Abamectin und Poloxamer mit Harz und Tocopherol, auf molekularer Basis zusammenschliessen und somit quasi einen Wirk-Hilfstoff-Komplex bilden. Ein einfaches Vermischen aller Komponenten mit anschliessendem Rühren führt zu einem Gehalt von maximal einigen Zehntel Prozenten des zugegebenen Pflanzenwirkstoffes. Der Schmelzpunkt von reinen Poloxameren liegt bei ca. 57°C bis 58°C. Diese Art der Solubilisierung, indem man in einer Schmelze also das Poloxamer, ein Harz bzw. ein Tocopherol als Hilfsstoff auf molekularer oder quasimolekularer Basis mit den zu solubilisierenden Pflanzenwirkstoffen zusammenbringt, erweist sich als Schlüssel zur Verbesserung der Wirkstoffbearbeitung.

Das grundsätzliche Verfahrensprinzip beinhaltet also breit formuliert, dass man zwei obligatorische Hilfsstoffe, nämlich Poloxamer und Harz bzw. Tocopherol mit einem fakultativen Lösungsmittel sowie dem Pflanzenwirkstoff vermischt und daraus eine Schmelze bei ca. 40°C bis 100°C erzeugt, wodurch die Protagonisten in einen innigen, quasi-molekularen Kontakt zueinander gebracht werden. Ein Poloxamer, namentlich etwa Poloxamer 188 und/oder Poloxamer 407 und/oder eines deren Substitute und/oder Derivate werden aufgeschmolzen und der zu behandelnde Pflanzenwirkstoff wird innig in dieser Schmelze dispergiert. Die Schmelz-Temperatur kann durch Zugabe von Lösungsmitteln erniedrigt werden. Bei diesen Lösungsmitteln kann es sich zum Beispiel um Wasser, Glycerin, Propylen-Glykol, Polyethylen-Glykol 400, Ethanol, Macrogol 400 oder Isopropanol handeln. Nach Einbringen des zu behandelnden Pflanzenwirkstoffes in die Schmelze wird dieselbe abgekühlt, bis sie spröde ist. Dies lässt sich durch Zugabe von Trockeneis beschleunigen. Dann wird diese erstarrte Schmelze in einer Mühle gemahlen, darin durch Messer zerhackt und zerschlagen und hernach mit Wasser in einer Emulgiermühle gewalzt, bis die Schmelzekristalle soweit zerkleinert sind, dass ihr Durchmesser 5 Mikrometer und weniger beträgt. Die Emulgiermühle weist vorteilhaft einen gekühlten Scherkopf auf, so dass die Schmelzekristalle sich beim Mahlen nicht über Gebühr erwärmen und ihre spröde Konsistenz bewahren. Trotz der Spröde des Materials laufen diese Vorgänge staubfrei ab. In dieser Emulgiermühle kann die zerkleinerte Schmelzesuspension das Zahnradsystem in einem Kreislauf mehrmals durchlaufen, wobei sie immer weiter zerkleinert wird bis sie eine hinreichende Feinheit erlangt hat. Diese kleinsten Kristalle bleiben thermostabil und auch durch weitere Zugabe von Wasser wird die Tensidoberfläche nicht "abgewaschen". Die Schmelzekristalle werden dann in Wasser gelöst oder dispergiert und dem Pflanzen-Erdreich zur Aufnahme durch die Pflanze über ihr Wurzelwerk beigegeben, oder die Lösung wird direkt auf den oberirdischen Teil der Pflanze gespritzt. Ein weiterer Vorteil ergibt sich aus der Stabilität der Mizellen. Wirkstoffe, die verfahrensgemäss als Solubilisat auf die Pflanzen gespritzt wurden und durch Verdunstung an deren Oberfläche angetrocknet sind, können nach der Ernte leicht mit Wasser von der Pflanzenoberfläche abgewaschen werden. Damit schmeckt zum Beispiel ein ungeschälter, bespritzter Apfel nach einem schnellen Wasserbad nicht mehr bitter.

Dank dieses Verfahrens lässt sich die Bioverfügbarkeit eines Pflanzenwirkstoffes wesentlich steigern, weil nun die Wirkstoffe auf molekularer Basis eingeschlossen sind und die Umhüllung von Poloxamer-Harz-Tocopherol auch bei noch so grosser Verdünnung in Wasser stabil bleibt (Quasi-Komplex). Werden verschiedene Pflanzenwirkstoff-Poloxamer-Harz-Tocopherol-Schmelzen in Wasser kombiniert, behält jedes Modul seine physikalischen Eigenschaften bei. Es findet praktisch keine Beeinflussung untereinander statt, sowie auch andere oberflächenaktive Tenside diese ,Komplexe' in Wasser nicht beinflussen. Grundsätzlich dient Poloxamer-Harz-Tocopherol in diesem Verfahren eingesetzt zum Solubilisieren und Dispergieren jedes lipophilen Pflanzenwirkstoffes.

Wie schon weiter oben beschrieben, haben Poloxamere in den verschiedenen Pharmacopoeas eine eigene Monografie, d.h. einen kompletten Wirkstoffbeschrieb. Die Poloxamere sind völlig inert. Sie dürfen auch in der Agro, der Kosmetik- und den Tierernährungsbranche benützt werden. Einzelne Poloxamere werden in parenteralen Heilmitteln eingesetzt. Poloxamere sind also unbedenklich.

Wird Poloxamer, Harz bzw. Tocopherol verfahrensgemäss eingesetzt, können lipophile und harzartige Pflanzenwirkstoffe in hydrophilem Milieu gelöst werden wie auch hydrophile Stoffe in lipophilem Milieu. Es können auch feste Teilchen in wässrigem Milieu solubilisiert und dispergiert werden. Eine Anwendung liegt zum Beispiel darin, Pollen in wässrigem Milieu zu lösen.

Folgende quantitativen Zusammensetzungen sind für Poloxamer-Schmelzen typisch: 0.1% bis 8% Pflanzenwirkstoff/e, ca. 10-20% ein Poloxamer und 1-15% ein natürliches oder ein künstlich zusammengestelltes Harz (oder anstelle davon ein Tocopherol). Solche Zusammensetzungen werden danach in Wasser solubilisiert oder suspendiert.

Feste oder halbfeste Pflanzenwirkstoffkonzentrate, die nach dem beschriebenen grundsätzlichen Verfahren durch Herstellen einer Wirkstoffschmelze gewonnen werden, wobei dieses Schmelzen zur Berührung von Hilfs- und Wirkstoff über eine grosse Oberfläche führt, wie dies sonst nur durch Sprüh- und/oder Gefriertrocknung erreicht wird, bieten für die Resorbtion der Wirkstoffe in den Pflanzen hervorragende Eigenschaften. Diese Eigenschaften sind unabhängig davon, ob die Wirkstoffkonzentrate final einer Desorbtion unterworfen werden oder nicht. Mit den oben beschriebenen Verfahren können daher feste Wirkstoffkonzentrate für Pflanzen hergestellt werden. Die Schmelzekristalle werden dann von der Pflanze mitsamt dem Wasser, in welchem sie solubilisiert sind, über ihr Wurzelwerk aufgenommen.

Explizit beinhaltet die Erfindung auch das Auftragen der Schmelze, wie sie oben beschrieben ist, an wasserlöslichen Makromolekülen, welche die Berührungsoberfläche des Poloxamer-Harz-Tocopherol-Wirkstoff-Komplexes noch weiter vergrössern sollen. Als geeignete hydrophile oder wasserlösliche Feststoffe mit grossen Oberflächen oder durch Sprüh- und/oder Gefriertrocknung vergrößerten Oberflächen (spezifische Oberfläche > 0.01 m²/g BET-Methode) sind besonders geeignet:
- Exudate, wie z.B. Gummi arabicum, Traganth, Karayagummi, Ghattigummi,
- Samenmehle, wie z.B. Guargummi, Johannisbrotkernmehl, Tarakernmehl, Tamarindengummi,
- Gerüstsubstanzen, wie z.B. Lärchengummi, Pektin, Agar, Alginat, Carrageen, Furcellaran,
- Biosynthetische Hydrokolloide, wie z.B. Xanthan,
- Modifizierte Hydrokolloide, wie z.B. Propylenglykolalginate, amidiertes Pektin,
- Cellulose-Derivate, wie z.B. Methylcellulose, Methylethylcellulose, Methylhydroxyethylcellulose, Methylhydroxypropylcellulose, Hydroxypropylcellulose, Natrium-Carboxymethylcellulose,
- Siliziumoxide, wie z.B. Aerosil,
- Proteine, wie z.B. Gelatine, Magermilchpulver,
- Zucker, wie Laktose, Mannit, Xylit, Sorbit, Dektran.

## Patentansprüche

1. Verfahren zum Solubilisieren, Dispergieren und Stabilisieren von Stoffen, ***dadurch gekennzeichnet*, dass** einerseits ein Poloxamer und andrerseits ein Harz und/oder ein Tocopherol zu einer kombinierten Schmelze aufgeschmolzen wird und der zu behandelnde Stoff ohne Zugabe von Wasser innig in dieser Schmelze dispergiert oder gelöst wird, sodass anschliessend in dieser Schmelze jederzeit durch Zugabe von beliebig viel Wasser stabile Mizellen erzeugbar sind, welche die Stoffe enthalten und/oder binden.

2. Verfahren zum Solubilisieren, Dispergieren und Stabilisieren von Stoffen nach Anspruch 1, **dadurch gekennzeichnet, daß** die erzeugte Schmelze zur Verhinderung einer Aushärtung mit gleichwarmen Wasser überdeckt wird und der sich **dadurch** spontan bildende Gel homogenisiert wird.

3. Verfahren zum Solubilisieren, Dispergieren und Stabilisieren von Stoffen nach Anspruch 1, ***dadurch gekennzeichnet,* dass** ein Poloxamer, namentlich etwa Poloxamer 188 und/oder Poloxamer 407 und/oder eines deren Substitute und/oder Derivate kombiniert mit einem Harz und/oder einem Tocopherol aufgeschmolzen wird und der zu behandelnde Stoff innig in dieser Schmelze dispergiert wird, wobei nach Einbringen des zu behandelnden Stoffes in die Schmelze dieselbe zur Verhinderung einer Aushärtung mit Wasser überzogen und die Schmelze homogenisiert wird.

4. Verfahren zum Solubilisieren, Dispergieren und Stabilisieren von Stoffen nach einem der vorangehenden Ansprüche, ***dadurch gekennzeichnet*, dass** ein Poloxamer kombiniert mit einem Harz und/oder einem Tocopherol bei der Schmelztemperatur aufgeschmolzen wird, die Temperatur der Schmelze durch Zugabe von Lösungsmitteln erniedrigt wird, der zu solubilisierende Stoff in der Schmelze dispergiert wird, die Schmelze zur Verhinderung einer Aushärtung mit Wasser der gleichen Temperatur überzogen wird, und dann die Schmelze homogenisiert wird, wodurch ein transparenter Gel als Solubilisierungsprodukt entsteht.

5. Verfahren zum Solubilisieren, Dispergieren und Stabilisieren von Stoffen nach einem der vorangehenden Ansprüche, ***dadurch gekennzeichnet,* dass** ein Poloxamer kombiniert mit einem Harz und/oder einem Tocopherol bei einer Temperatur von ca. 40-60°C aufgeschmolzen wird, die Temperatur der Schmelze durch Zugaben eines oder mehrerer Lösungsmittel aus der Auswahl Glyzerin, Propylen-Glykol, Polyethylen-Glykol 400, Ethanol, Macrogol 400, Isopropanol erniedrigt wird, dann der zu solubilisierende, allenfalls thermolabile Stoff, insbesondere Insulin, in der Schmelze dispergiert wird, die Schmelze zur Verhinderung einer Aushärtung mit Wasser der gleichen Temperatur überzogen wird, und dann die Schmelze homogenisiert wird, wodurch ein transparenter Gel als Solubilisierungsprodukt entsteht.

6. Verfahren zum Solubilisieren, Dispergieren und Stabilisieren von Stoffen nach Anspruch 1, ***dadurch gekennzeichnet,* dass** mindestens ein Poloxamer kombiniert mit einem Harz und/oder einem Tocopherol aufgeschmolzen werden und ein zu behandelnder Pflanzenwirkstoff innig in dieser Schmelze dispergiert oder gelöst wird, wobei dieselbe danach solange abgekühlt wird, bis sie spröde ist, dann in einer Mühle gemahlen und anschliessend mit Wasser in einem Emulgator nass gewalzt oder in einer Hammermühle nass zerschlagen wird, bis die Schmelzekristalle auf einen Durchmesser von weniger als 5 Mikrometer reduziert sind, wodurch eine Suspension erzeugt wird, die einer Pflanze oder dem Pflanzenerdreich zur Aufnahme beigebbar ist.

7. Verfahren zum Solubilisieren, Dispergieren und Stabilisieren von Stoffen nach Anspruch 6, ***dadurch gekennzeichnet,* dass** ein Poloxamer, namentlich etwa Poloxamer 188 und/oder Poloxamer 407 und/oder eines deren Substitute und/oder Derivate kombiniert mit einem Harz und/oder mit einem Tocopherol aufgeschmolzen werden und der zu behandelnde Pflanzenwirkstoff innig in dieser Schmelze dispergiert wird, wobei nach Einbringen des zu behandelnden Pflanzenwirkstoffes in die Schmelze dieselbe danach abgekühlt wird, bis sie spröde ist, und sie gemahlen und hernach auf einen Durchmesser von weiniger als 5 Mikrometer reduziert sind, die Schmelzekristalle dann in Wasser gelöst oder dispergiert werden, sodass die Lösung dem Pflanzen-Erdreich zur Aufnahme durch die Pflanze über ihr Wurzelwerk beigebbar ist oder direkt auf den oberirdischen Teil der Pflanze spritzbar ist.

8. Verfahren zum Solubilisieren, Dispergieren und Stabilisieren von Stoffen nach einem der Ansprüche 6 bis 7, ***dadurch gekennzeichnet,* dass** ein Poloxamer kombiniert mit einem Harz und/oder einem Tocopherol bei einer Temperatur von ca. 40-60°C aufgeschmolzen wird, die Temperatur der Schmelze durch Zugaben von Lösungsmittel erniedrigt wird, der zu solubilisierende Pflanzenwirkstoff in der Schmelze dispergiert wird, die Schmelze danach abgekühlt wird, bis sie spröde ist, und sie in einer Zerhacker-Mühle grob gemahlen und hernach mit Wasser in einer Hammer-oder Emulgiermühle nass feingemahlen wird, bis die Schmelzekristalle auf eine Durchmesser von weniger als 5 Mikrometer reduziert sind, die Schmelzekristalle dann in weiterem Wasser gelöst oder dispergiert werden, sodass die Lösung dem Pflanzen-Erdreich zur Aufnahme durch die Pflanze über ihr Wurzelwerk beigebbar ist oder direkt auf den oberirdischen Teil der Pflanze spritzbar ist.

9. Verfahren zum Solubilisieren, Dispergieren und Stabilisieren von Stoffen nach einem der Ansprüche 6 bis 8, ***dadurch gekennzeichnet,* dass** ein Poloxamer kombiniert mit einem Harz und/oder einem Tocopherol bei einer Temperatur von ca. 40-60°C aufgeschmolzen wird, die Temperatur der Schmelze durch Zugaben eines oder mehreren Lösungsmittel aus der. Auswahl Glycerin, Propylen-Glykol, Polyethylen-Glykol 400, Ethanol, Macrogol 400, Isopropanol erniedrigt wird, dann der zu solubilisierende, allenfalls thermolabile Pflanzenwirkstoff in der Schmelze dispergiert wird, die Schmelze danach abgekühlt wird, bis sie spröde wird, und sie in einer Zerhacker-Mühle grob gemahlen und hernach mit Wasser in einer Hammer-oder Emulgiermühle nass feingemahlen wird, bis die Schmelzekristalle auf einen Durchmesser von weniger als 5 Mikrometer reduziert sind, die Schmelzekristalle dann in weiterem Wasser gelöst oder dispergiert werden, sodass die Lösung dem Pflanzen-Erdreich zur Aufnahme durch die Pflanze über ihr Wurzelwerk beigebbar ist oder direkt auf den oberirdischen Teil der Pflanze spritzbar ist.

10. Stabilisiertes Erzeugnis, bestehend aus einer Schmelze oder einem transparenten Gel, erhältlich durch das Verfahren von Ansprüchen 1 bis 8, auf der Basis von wenigstens einem Poloxamer in Kombination mit einem natürlichen oder einem künstlich zusammengestellten Harz und/oder einem Tocopherol, sowie einem darin solubilisierten und stabilisierten Wirkstoff, in einer Konsistenz zwischen fest über halbfest, das heisst Aspik-artig bis hin zu flüssig.

11. Stabilisiertes Erzeugnis nach Anspruch 10 und erhältlich durch das Verfahren von Ansprüchen 2 bis 5, bestehend aus einem transparenten Gel in halbfester, das heisst aspik-artiger bis flüssiger Konsistenz, auf der Basis von wenigstens einem Poloxamer, namentlich Poloxamer 188 und/oder Poloxamer 407 und/oder eines deren Substitute und/oder Derivate in Kombination mit einem Harz und/oder einem Tocopherol, einem Lösungsmittel aus der Auswahl Glyzerin, Propylen-Glykol, Polyethylen-Glykol 400, Ethanol, Macrogol 400, Isopropanol sowie aus einem darin solubilisierten, dispergierten und stabilisierten lipohilen oder hydrophilen Wirkstoff.

12. Stabilisiertes Erzeugnis nach Anspruch 10 und erhältlich durch das Verfahren von Ansprüchen 2 bis 5, die Vitamine C, E und ACC enthaltend, in Form eines Shampoos mit der übrigen Zusammensetzung:
| | |
|---|---|
| Wasser | 74.11% |
| Sodium Laureth Sulfate | 11.67% |
| Cocamidopropyl Betain | 2.00% |
| Cocamide DEA | 1.70% |
| Disodium Laureth Sulfosuccinate | 1.32% |
| Parfum | 1.00% |
| **Poloxamer 407** | **1.40%** |
| **Poloxamer 188** | **0.60%** |
| Ascorbinsäure | 1.00% |
| Acetyl Cysteine | 1.00% |
| **Alfa-Tocopherole** | **0.50%** |
| Sodium Lauryl Sulfate | 0.80% |
| Phenoxyethanol | 0.50% |
| Imidazolidinyl Urea | 0.20% |
| PEG-120 Methyl Glucose Dioleate | 0.10% |
| Tetrasodium EDTA | 0.10% |

13. Stabilisiertes Erzeugnis nach Anspruch 10 und erhältlich durch das Verfahren von Ausprüchen 2 bis 5, in Form eines Gels, der Coenzym Q10, Vitamin C und Vitamin E enthält, mit der Zusammensetzung:
| | |
|---|---|
| Wasser | 71.43 % |
| **Poloxamer 188** | **8.93 %** |
| **Poloxamere 407** | **8.93 %** |
| **Alfa-Tocopherol** | **5.00 %** |
| Coenzym Q10 | 2.14% |
| Asccorbinsäure | 3.57% |

14. Stabilisiertes Erzeugnis nach Anspruch 10 und erhältlich durch das Verfahren von Ausprüchen 2 bis 5, in Form eines Gels, welcher Propolis enthält, mit folgender Zusammensetzung:
| | |
|---|---|
| Wasser | 70 % |
| **Poloxamer 188** | **18 %** |
| **Propolis** | **13 %** |

15. Stabilisiertes Erzeugnis in fester oder halbfester Form nach Anspruch 10, und erhältlich durch das Verfahren von Ausprüchen 2 bis 5, Wirkstoffkonzentrate enthaltend, dessen Schmelze oder Gel als wasserdampfbeladener und somit hydrophiler und/oder wasserlösliche Feststoff mit grosser Oberfläche wirkt, als Gelatine-Kapseln, als dosierte Pulversachets oder zu Tabletten und Brausetabletten komprimiet.

16. Stabilisiertes Erzeugnis in fester oder halbfester Form nach Anspruch 10, und erhältlich durch das Verfahren von Ausprüchen 2 bis 5, Wirkstoffkonzentrate enthaltend, dessen Schmelze oder Gel als wasserdampfbeladener und somit hydrophiler und/oder wasserlöslicher Feststoff mit grosser Oberfläche wirkt, als Gelatine-Kapseln oder zu Tabletten und Brausetabletten komprimiert, wobei die hydrophilen oder wasserlöslichen Feststoffe mit grossen Oberflächen oder die durch Sprüh- und/oder Gefriertrocknung vergrösserten Oberflächen (mit spezifischer Oberfläche > 0.01 m²/g BET-Methode) aus einem oder mehreren der folgenden Stoffe bestehen:
• Exudate, wie z.B. Gummi arabicum, Traganth, Karayagummi, Ghattigummi.
• Samenmehle, wie z.B. Guargummi, Johannisbrotkernmehl, Tarakernmehl, Tamarindengummi,
• Gerüstsubstanzen, wie z.B. Lärchengummi, Pektin, Agar, Alginat, Carrageen, Furcellaran,
• Biosynthetische Hydrokolloide, wie z.B. Xanthan,
• Modifizierte Hydrokolloide, wie z.B. Propylenglykolalginate, amidiertes Pektin,
• Cellulose-Derivate, wie z.B. Methylcellulose, Methylethylcellulose, Methylhydroxyethylcellulose, Methylhydroxypropylcellulose, Hydroxypropylcellu-lose, Natrium-Carboxymethylcellulose,
• Siliziumoxide, wie z.B. Aerosil,
• Proteine, wie z.B. Gelatine, Magermilchpulver,
• Zucker, wie Laktose, Mannit, Xylit, Sorbit, Dextran.

17. Stabilisiertes Erzeugnis nach Anspruch 10, und erhältlich durch das Verfahren von Ansprüchen 1 bis 5, enthaltend eines oder mehrere der folgenden lipophilen oder hydrophilen Stoffe, die geeignet sind für Ernährungsergänzungen, für Kosmetika und Dermatologika, nämlich Coenzym Q10, Vitamin C, Vitamin E, Betacarotin, Vitamin A, Vitamin D3, Lutein, Lycopene, Folsäure, Vitamin B12, Ω-3- und Ω-6-Fettsäuren.

18. Stabilisiertes Erzeugnis nach Anspruch 10, und erhältlich durch das Verfahren von Ansprüchen 1 bis 5, enthaltend eines odere mehrere der Stoffe Insulin, (Antidiabetika), Ciclosporin, Planzenextrakte aus Mariendistel, Passionsblume und Pestwurzel und deren Leitstoffe (wie etwa Silymarin, Chrysin etc.).

19. Stabilisiertes Erzeugnis nach Anspruch 10, und erhältlich durch das Verfahren von Ansprüchen 1 bis 5, Ansprüche 1 bis 5, enthaltend Polyphenylverbindungen mit einem Wirkstoffgehalt von 2-5% in wässrigem Milieu.

20. Stabilisiertes Erzeugnis nach Anspruch 10, und erhältlich durch das Verfahren von Ansprüchen 1 bis 5, enthaltend harzartige Stoffe gegen Infektionen oder für die Konservierung von Stoffen.

21. Stabilisiertes Erzeugnis nach Anspruch 10, und erhältlich durch das Verfahren von Ansprüchen 1 bis 5, enthaltend Stoffe für die Wundversorgung auf der Basis von Propolis. Selendioxid, Teeren, und/oder Steinölen.

22. Stabilisiertes Erzeugnis in Form eines Wirkstoffsubstrates für das Pflanzenwachstum, erhältlich durch das Verfahren von Ansprüche 1 bis 8, bestehend aus einer wasserlöslichen Suspension oder Lösung, enthaltend einen Wirkstoff. oder Schmelzepar-tikel eines Wirkstoffs in einer transparenten Mischung auf der Basis von wenigstens einem Poloxamer, einem Harz und/oder einem Tocopherol, wobei die Schmelzekristalle maximal 5 Mikrometer messen.

23. Stabilisiertes Erzeugnis in Form eines Wirkstoffsubstrates für das Pflanzenwachstum nach Anspruch 22, bestehend aus Schmelzekristallen auf der Basis von wenigstens einem Poloxamer, einem Harz und/oder einem Tocopherol und einem darin solubilisierten und stabilisierten Pflanzenwirkstoff, wobei die Schmelzekristalle 5 Mikrometer oder kleiner sind.

24. Stabilisiertes Erzeugnis in Form eines Wirkstoffsubstrates für das Pflanzenwachstum nach einem der Ansprüche 22 oder 23, bestehend aus Schmelzekristallen auf der Basis von wenigstens Poloxamer 188 und/oder Poloxamer 407 und/oder eines deren Substitute und/oder Derivate mit einem Harz und/oder einem Tocopherol, einem Lösungsmittel aus der Auswahl Glycerin, Propylen-Glykol, Polyethylen-Glykol 400, Ethanol, Macrogol 400, Isopropanol sowie aus einem darin solubilisierten, dispergierten und stabilisierten lipophilen oder hydrophilen Pflanzenwirkstoff.

25. Stabilisiertes Erzeugnis in Form eines Wirkstoffsubstrates für das Pflanzenwachstum nach einem der Ansprüche 22 bis 24, **dadurch gekennzeichnet, dass** es in hydrophile oder wasserlösliche Feststoffe mit grossen Oberflächen oder durch Sprüh- und/oder Gefriertrocknung vergrösserten Oberflächen (spezifische Oberfläche > 0.01 m2/g BET-Methode) gelöst ist, wobei diese Stoffe ein oder mehrere aus folgender Auswahl sind:
• Exudate, wie z.B. Gummi arabicum, Traganth, Karayagummi, Ghattigummi,
• Samenmehle, wie z.B. Guargummi, Johannisbrotkernmehl, Tarakernmehl, Tamarindengummi,
• Gerüstsubstanzen, wie z.B. Lärchengummi, Pektin, Agar, Alginat, Carrageen, Furcellaran,
• Biosynthetische Hydrokolloide, wie z.B. Xanthan,
• Modifizierte Hydrokolloide, wie z.B. Propylenglykolalginate, amidiertes Pektin,
• Cellulose-Derivate, wie z.B. Methylcellulose, Methylethylcellulose, Methylhydroxyethylcellulose, Methylhydroxypropylcellulose, Hydroxypropylcellulose, Natrium-Carboxymethylcellulose,
• Siliziumoxide, wie z.B. Aerosil,
• Proteine, wie z.B. Gelatine, Magermilchpulver,
• Zucker, wie Laktose, Mannit, Xylit, Sorbit, Dektran.

26. Verwendung eines Erzeugnisses nach Anspruch 10 oder 11 zum Lösen von Fetten, Eiweissen, Harzen und harzartigen Stoffe aller Art, zum Ablösen von Fetten, Eiweissen, Harzen und harzartigen Stoffe aller Art von Oberflächen.

27. Verwendung eines Erzeugnisses nach Anspruch 10 oder 11 zum Pflegen und Reinigen von Gegenständen.

28. Verwendung eines Erzeugnisses nach Anspruch 10 oder 11 als Schmiermittel.

29. Verwendung eines Erzeugnisses nach Anspruch 10 oder 11 zum Maskieren eines Stoffes zur Unterdrückung dessen Geruchs- und/oder Geschmacksentfaltung.

30. Verwendung eines stabilisierten Erzeugnisses in Form eines Wirkstoffsubstrates, hergestellt nach dem Verfahren gemäss einem der Ansprüche 22 bis 25, in Form einer Suspension oder Lösung, enthaltend einen Wirkstoff oder Schmelzepartikel eines Wirkstoffs in einer transparenten Mischung auf der Basis von wenigstens einem Poloxamer, einem Harz und/oder einem Tocopherol, wobei die Schmelzekristalle maximal 5 Mikrometer messen, zum Beeinflussen des Pflanzenwachstums, indem die Suspension oder Lösung in Wasser gelöst oder suspendiert dem Pflanzen-Erdreich zur Aufnahme über das Wurzelwerk beigegeben wird, oder der oberirdische Teil der Pflanze damit direkt bespritzt wird.

31. Verwendung eines stabilisierten Erzeugnisses in Form eines Wirkstoffsubstrates nach einem der Ansprüche 22 bis 25 zum Beeinflussen des Pflanzenwachstums, indem er in Wasser gelöst dem Pflanzen-Erdreich zur Aufnahme über das Wurzelwerk beigegeben wird, oder direkt auf den oberirdischen Teil der Pflanze gespritzt wird.

## Claims

1. Method for solubilising, dispersing and stabilising substances, ***characterised in that*** on one hand a poloxamer and on the other a resin and/or a tocopherol is melted to a combined molten mass and the substance to be treated is solubilised or dispersed within this molten mass without the addition of water, so that later on by adding water to this molten mass any number of water-stable micelles can be produced, which retain and/or bind the substances.

2. Method for solubilising, dispersing and stabilising substances according to the claim 1, **characterised in that** the generated molten mass is covered with a coating of water at the same temperature for preventing a hardening and the gel that thus forms spontaneously is homogenised.

3. Method for solubilising, dispersing and stabilising substances according to the claim 1, ***characterised in* that** a poloxamer, namely Poloxamer 188 and/or Poloxamer 407 and/or one of their substitutes and/or derivatives combined with a resin and/or a tocopherol are melted together and the substance to be treated is dispersed inside this molten mass, so that after introducing the substance to be treated the same is coated with water for preventing a hardening and the molten mass is homogenised.

4. Method for solubilising, dispersing and stabilising substances according to one of the earlier claims, ***characterised in* that** a poloxamer combined with a resin and/or a tocopherol are melted at the melting point, the temperature of the molten mass is lowered by adding a solvent, the substance to be solubilised is dispersed in the molten mass, the molten mass is coated with water at the same temperature for preventing a hardening, and then the molten mass is homogenised, which gives rise to a transparent gel as the solubilisation product.

5. Method for solubilising, dispersing and stabilising substances according to one of the earlier claims, ***characterised in* that** a poloxamer combined with a resin and/or a tocopherol is melted at a temperature of approx. 40-60°C, the temperature of the molten mass is lowered by the addition of one or more solvents from a selection of water, glycerine, propylene glycol, polyethylene glycol 400, ethanol, Macrogol 400, isopropanol, then the substance to be solubilised, possibly thermo-labile, especially insulin, is dispersed in the molten mass, the molten mass is coated with water at the same temperature for preventing a hardening, and then the molten mass is homogenised, which gives rise to a transparent gel as the solubilisation product.

6. Method for solubilising, dispersing and stabilising substances according to the claim 1, ***characterised in* that** at least one poloxamer combined with a resin and/or a tocopherol is melted and an active agent for plants to be treated is dispersed or dissolved inside this molten mass, then this is cooled till it becomes brittle, then milled and thereafter wet rolled with water in an emulsifier or shattered wet in a hammer mill, till the melt crystals are reduced to a diameter of less than 5 micrometer, which gives rise to a suspension, which is added to a plant or in the humus area of a plant for the absorption.

7. Method for solubilising, dispersing and stabilising substances according to the claim 6, ***characterised in* that** at least one poloxamer, namely Poloxamer 188 and/or Poloxamer 407 and/or one of their substitutes and/or derivatives combined with a resin and/or a tocopherol are melted together and the active agent for plants to be treated is dispersed inside this molten mass, so that after adding, this is then cooled afterwards for so long, till it becomes brittle, is then milled and then reduced to a diameter of less than 5 micrometer, the melt crystals are then dissolved or dispersed in water, so that the solution can be poured in the ground for absorption by the plant through its root network or can be spayed directly on to the part of the plant above the ground.

8. Method for solubilising, dispersing and stabilising substances according to one of the claims 6 to 7, ***characterised in* that that** a poloxamer combined with a resin and/or a tocopherol is melted at a temperature of approx. 40-60°C, the temperature of the molten mass is lowered by the addition of one or more solvents, the active agent for plants to be solubilised is dispersed in the molten mass, the molten mass is thereafter cooled for so long, till it is brittle, and is milled coarsely in a chopper mill and then wet fine-milled with water in a hammer or an emulsion mill, till the melt crystals have been reduced to a diameter of less than 5 micrometer, the melt crystals are then dissolved or dispersed further in water, so that the solution can be added directly in the humus area of the plant in its root network or can be sprayed directly on to the part of the plant above the ground.

9. Method for solubilising, dispersing and stabilising substances according to one of the claims 6 to 8, ***characterised in* that that** a poloxamer combined with a resin and/or a tocopherol is melted at a temperature of approx. 40-60°C, the temperature of the molten mass is lowered by the addition of one or more solvents from a selection of water, glycerine, propylene glycol, polyethylene glycol 400, ethanol, Macrogol 400, isopropanol, then the substance to be solubilised, possibly thermo-labile active agent for plants is dispersed in the molten mass, the molten mass is then cooled till it becomes brittle, and is then milled coarsely in a chopper mill, then wet fine-milled with water in a hammer or an emulsion mill, till the melt crystals have been reduced to a diameter of less than 5 micrometer, the melt crystals are then dissolved or dispersed further in water, so that the solution can be added directly in the humus area of the plant in its roots or can be sprayed directly on to the part of the plant above the ground.

10. Stabilised product, comprising of a molten mass or a transparent gel, obtainable by the method of claims 1 to 8, on the basis of at least one poloxamer in combination with a natural or an artificially prepared resin and/or a tocopherol, as well as an active agent solubilised in it and stabilised, in a consistency between solid and semi-solid, i.e. aspic-like to a fluid.

11. Stabilised product according to the claim 10, obtainable by the method of claims 2 to 5, comprising of a transparent gel in semi-solid, i.e. aspic-like to a fluid consistency, on the basis of at least one poloxamer, namely Poloxamer 188 and/or Poloxamer 407 and/or one of their substitutes and/or derivates in combination with a resin and/or a tocopherol, a solvent from a selection of water, glycerine, propylene glycol, polyethylene glycol 400, ethanol, Macrogol 400, isopropanol as well as a lipophilic or hydrophilic active agent solubilised, dispersed or stabilised in it.

12. Stabilised product according to the claim 10 and obtainable by the method of claims 2 to 5, containing the vitamins C, E and ACC, in the form of a shampoo with the remaining composition as:
• Water 74.11 %
• Sodium Laureth Sulphate 11.67%
• Cocamidopropyl Betain 2.00%
• Cocamide DEA 1.70%
• Disodium Laureth Sulphosuccinate 1.32%
• Perfume 1.00%
• **Poloxamer 407 1.40%**
• **Poloxamer 188 0.60%**
• Ascorbic acid 1.00%
• Acetyl Cysteine 1.00%
• **Alpha tocopherols 0.50%**
• Sodium Lauryl Sulphate 0.80%
• Phenoxyethanol 0.50%
• Imidazolidinyl Urea 0.20%
• PEG-120 Methyl Glucose Dioleate 0.10%
• Tetra sodium EDTA 0.10 %

13. Stabilised product according to the claim 10, obtainable by the method of claims 2 to 5 in the form of a gel, containing the coenzyme Q10, vitamin C and vitamin E, with the composition:
• Water 71.43%
• **Poloxamer 188 8.93%**
• **Poloxamers 407 8.93%**
• **Alpha tocopherol 5.00%**
• Coenzyme Q10 2.14%
• Ascorbic acid 3.57%

14. Stabilised product according to the claim 10, obtainable by the method of claims 2 to 5 in the form of a gel, which contains propolis, with the following composition:
• Water 70%
• **Poloxamer 188 18%**
• **Propolis 13%**

15. Stabilised product in solid or semi-solid form according to the claim 10, obtainable by the method of claims 2 to 5, containing the concentrate of the active agent, whose molten mass or gel acts a solid charged with water vapour and thus hydrophilic and/or as water-soluble solid with a large surface, compressed as gelatine capsules, as dosed powder sachets or as tablets and effervescent tablets.

16. Stabilised product in solid or semi-solid form according to the claim 10, obtainable by the method of claims 2 to 5, containing the concentrate of the active agent, whose molten mass or gel acts a solid charged with water vapour and thus hydrophilic and/or as water-soluble solid with a large surface, compressed as gelatine capsules, as dosed powder sachets or as tablets and effervescent tablets, such that they are hydrophilic or water-soluble solids with large surface or with surface enlarged through spray and/or freeze drying (with specific surface > 0.01 m²/g BET method) comprising of one or more of the following substances:
• Exudates, such as gum arabic, tragacanth, karaya gum, ghatti gum,
• Seed flours, such as guar gum, carob bean flour, tara stone flour, tamarind gum,
• Detergent builders, such as larch gum, pectin, agar, alginate, carrageen, furcellaran,
• Bio-synthetic hydrocolloids, such as xanthan,
• Modified hydrocolloids, such as propylene glycol alginate, amidated pectin,
• Cellulose derivatives, such as methyl cellulose, methyl ethyl cellulose, methyl hydroxyl ethyl cellulose, methyl hydroxyl propyl cellulose, hydroxyl propyl cellulose, sodium carboxy-methyl cellulose,
• Silicon oxides, such as Aerosil,
• Proteins, such as gelatine, skimmed milk powder,
• Sugars, such as lactose, mannitol, xylite, sorbitol, dextran.

17. Stabilised product according to the claim 10, obtainable by the method of claims 1 to 5, containing one or more of the following lipophilic or hydrophilic substances, which are suitable for food supplements, for cosmetics and dermatological products, namely coenzyme Q10, vitamin C, vitamin E, beta-carotene, vitamin A, vitamin D3, lutein, lycopene, folic acid, vitamin B12, Ω-3 and Ω-6 fatty acids.

18. Stabilised product according to the claim 10, obtainable by the method of claims 1 to 5, containing one or more of the substances such as insulin, (antidiabetic), cyclosporine, plant extracts from holy thistle, passion flower and butterbur, etc. and their derivatives (such as Silymarin, Chrysin etc.).

19. Stabilised product according to the claim 10, obtainable by the method of claims 1 to 5, containing polyphenyl compounds with an active agent content of 2-5% in aqueous milieu.

20. Stabilised product according to the claim 10, obtainable by the method of claims 1 to 5, containing resin-like substances against infections or for preservation of substances.

21. Stabilised product according to the claim 10, obtainable by the method of claims 1 to 5, containing substances for wound dressing on the basis of propolis, selenium dioxide, tars and/or mineral oils.

22. Stabilised product in the form of a concentrate of the active agent for the growth of the plant, obtainable by the method of claims 1 to 8, comprising of a water-soluble suspension or solution, containing an active agent or melt particles of an active agent in a transparent mixture on the basis of at least one poloxamer, a resin and/or a tocopherol, where the melt crystals measure maximum 5 micrometer.

23. Stabilised product in the form of a concentrate of the active agent for the growth of the plant according to the claim 22, comprising of melt crystals on the basis of at least a poloxamer, a resin and/or a tocopherol and an active agent for plants solubilised and stabilised in it, where the melt crystals are 5 micrometer or smaller.

24. Stabilised product in the form of a concentrate of the active agent for the growth of the plant according to the claim 22 or 23, comprising of melt crystals on the basis of at least poloxamer 188 and/or poloxamer 407 and/or one of their substitutes and/or derivatives with a resin and/or a tocopherol, a solvent from the selection of water, glycerine, propylene glycol, polyethylene glycol 400, ethanol, Macrogol 400, isopropanol as well as a lipophilic or hydrophilic active agent for plants solubilised, dispersed or stabilised in it.

25. Stabilised product in the form of a concentrate of the active agent for the growth of the plant according to the claim 22 or 24, **characterised in that** it is dissolved in hydrophilic or water-soluble solids with large surface or with surface enlarged through spray and/or freeze-drying (specific surface > 0.01 m²/g BET method), where these substances are one or more from the following selection:
• Exudates, such as gum arabic, tragacanth, karaya gum, ghatti gum,
• Seed flours, such as guar gum, carob bean flour, tara stome flour, tamarind gum,
• Detergent builders, such as larch gum, pectin, agar, alginate, carrageen, furcellaran,
• Bio-synthetic hydrocolloids, such as xanthan,
• Modified hydrocolloids, such as propylene glycol alginate, amidated pectin,
• Cellulose derivatives, such as methyl cellulose, methyl ethyl cellulose, methyl hydroxyl ethyl cellulose, methyl hydroxyl propyl cellulose, hydroxyl propyl cellulose, sodium carboxy-methyl cellulose,
• Silicon oxides, such as Aerosil,
• Proteins, such as gelatine, skimmed milk powder,
• Sugars, such as lactose, mannitol, xylite, sorbitol, dextran.

26. Use of a product according to the claim 10 or 11 for dissolving fats, proteins, resins and resin-like substances of all kinds, for releasing fats, proteins, resins and resin-like substances of all kinds from the surfaces.

27. Use of a product according to the claim 10 or 11 for maintenance and cleaning of objects.

28. Use of a product according to the claim 10 or 11 as lubricant.

29. Use of a product according to the claim 10 or 11 for sequestering a substance for suppressing its smell and/or taste.

30. Use of a stabilised product in the form of a concentrate of an active agent, produced according to the method according to one of the claims 22 to 25, in the form of a suspension or solution, containing an active agent or melt particles of an active agent in a transparent mixture on the basis of at least a poloxamer, a resin and/or a tocopherol, where the melt crystals measure a maximum of 5 micrometer, for influencing the growth of the plant, by adding the suspension or the solution in water in a dissolved or suspended state in the humus area of the plant for absorption, or by directly spraying it on to the part of the plant above the soil.

31. Use of a stabilised product in the form of a concentrate of an active agent according to one of the claims 22 to 25 for influencing the growth of the plant, by adding the suspension or the solution in water in a dissolved state in the root area of the plant for absorption, or by directly spraying it on to the part of the plant above the soil.

## Revendications

1. Dispositif pour solubiliser, disperser et stabiliser des produits, **caractérisé en ce que** d'une part, un poloxamère et d'autre part, une résine et ou un tocophérol sont fondus dans une fusion combinée, et **en ce que** le produit à traiter est dispersé ou dissout intimement sans ajout d'eau dans cette fusion pour pouvoir fabriquer à tout moment après, dans cette fusion, des cellules de micelles stables en ajoutant beaucoup d'eau, lesquelles cellules renferment les matériaux et ou les lient.

2. Dispositif pour solubiliser, disperser et stabiliser des produits selon la revendication 1, **caractérisé en ce que** la fusion produite pour empêcher un durcissement est recouverte d'une eau d'une même température et **en ce que** le gel qui se forme ainsi spontanément se trouve homogénéisé.

3. Dispositif pour solubiliser, disperser et stabiliser des produits selon la revendication 1, **caractérisé en ce qu'**un poloxamère, notamment par exemple un poloxamère 188 et/ou un poloxamère 407 et/ou un de ses substituts et/ou dérivés, combinés à une résine et/ou un tocophérol, est fondu et, **en ce que** la matière à traiter est dispersée dans cette fusion; où, après l'ajout de la matière à traiter dans la fusion, celle-ci est recouverte d'eau pour empêcher un durcissement, et la fusion est homogénéisée.

4. Dispositif pour solubiliser, disperser et stabiliser des produits selon une des revendications précédentes, **caractérisé en ce qu'**un poloxamère qui est combiné avec une résine et/ou un tocophérol est fondu lors de la température de fusion, **en ce que** la température de la fusion est abaissée par l'ajout de solvants, **en ce que** la matière à solubiliser est dispersée dans la fusion, **en ce que** la fusion est soumise à la même température pour empêcher un durcissement au contact de l'eau, **en ce qu'**ensuite la fusion est homogénéisée; le résultat obtenu étant un gel transparent comme produit de solubilisation.

5. Dispositif pour solubiliser, disperser et stabiliser des produits selon une des revendications précédentes, **caractérisé en ce qu'**un poloxamère qui est combiné avec une résine et/ou un tocophérol à une température d'environ 40-60°C est fondu, **en ce que** la température de la fusion est abaissée par l'ajout d'un ou de plusieurs solvants choisis parmi la glycérine, propylène glycol, polyéthylène glycol 400, de l'éthanol, macrogol 400, isopropanol, puis la matière à solubiliser, à la rigueur thermolabile, notamment de l'insuline, est dispersée dans la fusion, **en ce que** la fusion est soumise à la même température pour empêcher un durcissement au contact de l'eau, **en ce qu'**ensuite la fusion est homogénéisée; le résultat obtenu étant un gel transparent comme produit de solubilisation.

6. Dispositif pour solubiliser, disperser et stabiliser des produits selon la revendication 1, **caractérisé en ce qu'**un poloxamère qui est combiné avec une résine et/ou un tocophérol sont fondus et **en ce qu'**un agent actif matière végétale de traitement est dispersée intimement dans cette fusion ou dissoute, laquelle fusion est refroidie ensuite jusqu'à ce qu'elle soit cassante, puis moulue dans un moulin et passée ensuite dans un émulseur de façon mouillée au rouleau avec de l'eau ou cassée de façon mouillée dans un moulin à marteau jusqu'à ce que les cristaux de fusion soient réduits à un diamètre inférieur à 5 micromètres; le résultat étant une suspension qui est versée à la plante ou à la terre de la plante pour y être absorbée.

7. Dispositif pour solubiliser, disperser et stabiliser des produits selon la revendication 6, **caractérisé en ce qu'**un poloxamére notamment par exemple un poloxamère 188 et/ou un poloxamère 407 et/ou un de ses substituts et/ou dérivés qui sont combinés avec une résine et/ou un tocophérol sont fondus et la matière végétale à traiter est dispersée intimement dans cette fusion; ladite fusion étant refroidie jusqu'à ce qu'elle soit cassante après l'ajout de la matière végétale à traiter, et moulue, puis réduite à un diamètre inférieur à 5 micromètres; les cristaux de fusion étant alors dissouts dans de l'eau et dispersés jusqu'à ce que la solution puisse être versée dans la terre de la plante et être absorbée par ses racines, ou puisse être aspergée directement sur la partie hors du sol de la plante.

8. Dispositif pour solubiliser, disperser et stabiliser des produits selon une des revendications 6 à 7, **caractérisé en ce qu'**un poloxamére qui est combiné avec une résine et/ou un tocophérol à une température d'environ 40-60°C est fondu, **en ce que** la température de la fusion est abaissée par l'ajout d'un ou de plusieurs solvants, **en ce que** la matière végétale à solubiliser est répandue dans la fusion, puis **en ce que** la fusion est refroidie jusqu'à être cassante, et grossièrement moulue dans un moulin hacheur et ensuite moulue finement mouillée avec de l'eau dans un moulin à émulsifier ou à marteau jusqu'à ce que les cristaux de fusion soient réduits à un diamètre inférieur à 5 micromètres, et que les cristaux de fusion soient ensuite dissouts dans de l'eau ou dispersés pour que la solution puisse être versée dans la terre de la plante et y être absorbée par ses racines ou être directement aspergée sur la partie hors de terre de la plante.

9. Dispositif pour solubiliser, disperser et stabiliser des produits selon la revendication 6 à 8, **caractérisé en ce qu'**un poloxamére qui est combiné avec une résine et/ou un tocophérol à une température d'environ 40-60°C soient fondus, que la température de la fusion est abaissée par l'ajout d'un ou de plusieurs solvants choisis parmi la glycérine, propylène glycol, polyéthylène glycol 400, de l'éthanol, macrogol 400, isopropanol, puis la matière à solubiliser, à la rigueur thermolabile, notamment de l'insuline, est dispersée dans la fusion, **en ce que** la fusion est ensuite refroidie jusqu'à ce qu'elle soit cassante, et grossièrement moulue dans un moulin hacheur, puis moulue finement mouillée avec de l'eau dans un moulin à émulsifier ou à marteau jusqu'à ce que les cristaux de fusion soient réduits à un diamètre inférieur à 5 micromètres; les cristaux de fusion étant alors dissouts dans de l'eau ou dispersés jusqu'à ce que la solution soit versée dans la terre de la plante pour y être absorbée par ses racines ou être directement aspergée sur la partie hors de terre de la plante.

10. Résultat stabilisé, composé d'une fusion ou d'un gel transparent obtenu par le dispositif selon les revendications 1 à 8, fabriqué selon une des revendication 1 à 2 sur la base d'au moins un poloxamére qui est combiné avec une résine naturelle ou synthétique et/ou un tocophérol ainsi qu'une matière solubilisée dedans et stabilisée avec une consistance se situant entre le solide à mi-solide, c'est-à-dire, sous une forme d'aspic jusqu'à liquide.

11. Résultat stabilisé selon la revendication 10 et que l'on peut obtenir grâce au dispositif des revendications 1 à 5, composé d'un gel transparent, d'une consistance mi-solide, c'est-à-dire, sous forme d'aspic jusqu'à liquide, sur la base d'au moins un poloxamére, à savoir un poloxamére 188 et/ou un poloxamére 407 et/ou un de leurs substituts ou dérivés, combinés avec une résine et/ou un tocophérol, d'un solvant parmi lesquels : la glycérine, propylène glycol, polyéthylène glycol 400, de l'éthanol, macrogol 400, isopropanol, ainsi qu'une matière lipophile ou hydrophile, solubilisée, dispersée dedans.

12. Résultat stabilisé selon la revendication 10 et que l'on peut obtenir grâce au dispositif selon les revendications 2 à 5, contenant de la vitamine C, E et ACC, sous la forme d'un shampooing avec la composition suivante :
| | |
|---|---|
| Eau | 74.11% |
| Sodium Laureth Sulfate | 11.67% |
| Cocamidopropyl Betain | 2.00% |
| Cocamide DEA | 1.70% |
| Disodium Laureth Sulfosuccinate | 1.32% |
| Parfum | 1.00% |
| **Poloxamére 407** | **1.40 %** |
| **Poloxamére 188** | **0.60 %** |
| acide ascorbique | 1.00% |
| Acétyle Cystéine | 1.00% |
| **Alfa-Tocophérol** | **0.50 %** |
| Sodium Lauryl Sulfate | 0.80% |
| Phénoxyéthanol | 0.50% |
| urée d'imidazolidinyle | 0.20% |
| PEG-120 Méthyle Glucose Dioléat | 0.10% |
| Tétra sodium EDTA | 0.10% |

13. Résultat stabilisé selon la revendication 10 et que l'on peut obtenir grâce au dispositif selon les revendications 2 à 5, sous la forme d'un gel contenant du Coenzyme Q10, de la vitamine C et de la vitamine E, avec la composition suivante :
| | |
|---|---|
| Eau | 71.43% |
| **Poloxamére 188** | **8.93 %** |
| **Poloxamére 407** | **8.93 %** |
| **Alfa-Tocophérol** | **5.00 %** |
| Coenzyme Q1 | 2.14% |
| Acide Ascorbique | 3.57% |

14. Résultat stabilisé selon la revendication 10 et que l'on peut obtenir grâce au dispositif selon les revendications 2 à 5, sous la forme d'un gel contenant de la Propolis, avec la composition suivante :
| | |
|---|---|
| Eau | 70% |
| **Poloxamére 188** | **18 %** |
| **Propolis** | **13 %** |

15. Résultat stabilisé sous une forme solide ou mi-solide selon la revendication 10 et que l'on peut obtenir grâce au dispositif des revendications 2 à 5, comprenant des concentrés de produits actifs dont la fusion ou le gel agit comme matériau solide à large surface, chargé de vapeur d'eau et donc hydrophile et/ou soluble dans l'eau, comme capsules de gélatine, comme sachets de poudre dosés ou comprimés sous forme de cachets et de cachets solubles.

16. Résultat stabilisé sous une forme solide ou mi-solide selon la revendication 10 et que l'on peut obtenir grâce au dispositif des revendications 2 à 5, comprenant des concentrés de produits actifs dont la fusion ou le gel agit comme matériau solide à large surface, chargé de vapeur d'eau et donc hydrophile et/ou soluble dans l'eau, comme capsules de gélatine ou comme cachets et cachets solubles comprimés; les matériaux solides hydrophiles ou solubles dans l'eau à large surface ou les surfaces agrandies via séchage par vaporisation ou refroidissement (avec une surface spécifique > 0.01 m²/g procédé BET) étant constitués d'un ou de plusieurs matières suivantes:
• Exudates, comme par exemple, de la gomme arabique, la gomme de traganthe, la gomme karaya, la gomme ghatti,
• Farines de graines, comme par exemple, la gomme guar, la farine de grains de caroube, la farine de graines de tara, gomme d'écorce de tama,
• substances d'ossature, comme par exemple, la gomme de mélèze, pectine, agar, alginat, carrageen, furcellaran,
• hydrocolloïdes biosynthétiques, comme par exemple, le xanthan,
• hydrocolloïdes modifiés, comme par exemple, le propylèneglycolalginate, la pectine amidée,
• les dérivés de la cellulose, comme par exemple, la cellulose méthylique, la méthyléthylcellulose, la méthylhydroxyéthylcellulose, la méthylhydroxypropylcellulose, la hydroxypropylcellulose, la carboxyméthylcellulose de sodium,
• l'oxide de silice, comme par exemple, 'aerosile,
• Des protéines, comme par exemple, de la gélatine, de la poudre de lait écrémé,
• Du sucre, comme du lactose, mannit, xylit, sorbit, dextran.

17. Résultat stabilisé selon la revendication 10, et que l'on peut obtenir grâce au dispositif des revendications 1 à 5, comprenant un ou plusieurs des matériaux lipophyles ou hydrophiles suivants qui sont appropriés en tant que compléments alimentaires, pour les cosmétiques et la dermatologie, notamment le coenzyme Q10, la vitamine C, la vitamine E, le bétacarotène, la vitamine A, la vitamine D3, la lutéine, lycopène, l'acide folique, la vitamine B12, les acides aminés Ω-3- et Ω-6.

18. Résultat stabilisé selon la revendication 10, et que l'on peut obtenir grâce au dispositif des revendications 1 à 5, comprenant un ou plusieurs des matériaux suivants : insuline, (Anti-diabétique), Ciclosporine, extraits de plantes de chardon - Marie, Fleurs de la Passion et la racine de pas d'ane et leurs matières de transport (comme par exemple silymarin, chrysin etc.).

19. Résultat stabilisé selon la revendication 10, et que l'on peut obtenir grâce au dispositif des revendications 1 à 5, comprenant des composés de polyphényle avec un taux de produit actif de 2-5% dans un milieu aqueux.

20. Résultat stabilisé selon la revendication 10, et que l'on peut obtenir grâce au dispositif des revendications 1 à 5, comprenant des matériaux à base de résine contre les infections ou pour conserver des matières.

21. Résultat stabilisé selon la revendication 10, et que l'on peut obtenir grâce au dispositif des revendications 1 à 5, comprenant des matériaux pour soigner des plaies à base de propolis, de dioxyde de sélène, des goudrons, et/ou des pétroles .

22. Résultat stabilisé sous la forme d'un substrat à produit actif pour faire pousser les plantes, que l'on peut obtenir grâce au dispositif des revendications 1 à 8, composé d'une suspension soluble dans l'eau ou d'une solution comprenant un produit actif ou des particules de la fonte d'un agent dans un mélange transparent, sur la base d'au moins un poloxamère, d'une résine et/ou d'un tocophérol; les cristaux de fusion mesurant au maximum 5 micromètres.

23. Résultat stabilisé sous la forme d'un substrat à produit actif pour faire pousser les plantes selon la revendication 22, composé de cristaux de fusion sur la base d'au moins un poloxamère, d'une résine et/ou d'un tocophérol et d'un produit actif végétal solubilisé et stabilisé dedans; les cristaux de fusion ayant une taille de 4 micromètres ou moins.

24. Résultat stabilisé sous la forme d'un substat de produit actif pour faire pousser les plantes selon les revendications 22 ou 23, composé de cristaux de fusion sur la base d'au moins un poloxamère 188 et/ou un poloxamère 407 et/ou un de leurs substituts et/ou dérivés avec une résine et/ou un tocophérol, un solvant choisis parmi la glycérine, propylène glycol, polyéthylène glycol 400, l'éthanol, macrogol 400, isopropanol ainsi qu'une matière végétale lipophile ou hydrophile, solubilisée et dispersée dedans.

25. Résultat stabilisé sous la forme d'un substrat de produit actif pour faire pousser les plantes selon les revendications 22 ou 23, **caractérisé en ce qu'**il est dissout dans des matières solides hydrophiles ou solubles dans l'eau à larges surfaces ou via des surfaces agrandies par un séchage par vaporisation et/ou congélation (surface spécifique > 0.01 m2/g procédé BET); ces matériaux étant choisis parmi un ou plusieurs des composants suivants :
• Exudates, comme par exemple, de la gomme arabique, la gomme de traganthe, la gomme karaya, la gomme ghatti,
• Farines de graines, comme par exemple, la gomme guar, la farine de grains de caroube, la farine de graines de tara, gomme d'écorce de tama,
• substances d'ossature, comme par exemple, la gomme de mélèze, pectine, agar, alginat, carrageen, furcellaran,
• hydrocolloïdes biosynthétiques, comme par exemple, le xanthan,
• hydrocolloïdes modifiés, comme par exemple, le propylèneglycolalginate, la pectine amidée,
• les dérivés de la cellulose, comme par exemple, la cellulose méthylique, la méthyléthylcellulose, la méthylhydroxyéthylcellulose, la méthylhydroxypropylcellulose, la hydroxypropylcellulose, la carboxyméthylcellulose de sodium,
• □ l'oxide de silice, comme par exemple, 'aerosile,
• Des protéines, comme par exemple, de la gélatine, de la poudre de lait écrémé,
• Du sucre, comme du lactose, mannit, xylit, sorbit, dextran.

26. Utilisation d'un résultat selon la revendication 10 ou 11 pour dissoudre des graisses, des protéines, des résines et des matériaux à base de résine de tout type, pour remplacer des graisses, protéines, résines et matériaux à base de résine de tout type de surfaces.

27. Utilisation d'un résultat selon la revendication 10 ou 11 pour entretenir et nettoyer des objets.

28. Utilisation d'un résultat selon la revendication 10 ou 11 comme lubrifiant .

29. Utilisation d'un résultat selon la revendication 10 ou 11 pour masquer une matière en étouffant son odeur et/ou son goût.

30. Utilisation d'un résultat sous la forme d'un substrat à produit actif, fabriqué selon le dispositif conforme à une des revendications 22 à 25, sous la forme d'une suspension ou d'une solution comprenant un produit actif/une matière active ou une particule de fusion d'un produit actif/d'une matière active, dans un mélange transparent sur la base d'au moins un poloxamère, d'une résine et/ou d'un Tocophérol; les cristaux de fusion mesurant maximum 5 micromètres, pour influencer la pousse d'une plante, en ce que la suspension ou la solution est dissoute dans de l'eau ou donnée sous forme suspendue à la terre de la plante pour que ses racines l'absorbe, ou en ce que la partie hors de terre de la plante soit directement aspergée par ladite suspension ou solution.

31. Utilisation d'un résultat sous la forme d'un substrat à produit actif selon une des revendications 22 à 25, pour influencer la pousse de la plante, en ce que ledit substrat est dissout dans l'eau et versé à la terre de la plante pour que ses racines l'absorbe, ou directement aspergé sur la partie hors de terre de la plante.
